# EUROPEAN PATENT SPECIFICATION

(11) **EP 3 687 591 B1**
(45) Date of publication and mention of the grant of the patent: **11.11.2026**
(21) Application number: 18782896.7
(22) Date of filing: 24.09.2018
(51) Int. Cl.: A61M 1/00, G16H 40/67, G16H 10/60

(54) **DEVICE OPERATION MONITORING AND CONTROL IN WOUND THERAPY SYSTEMS**
ÜBERWACHUNG UND STEUERUNG DES GERÄTEBETRIEBS BEI WUNDTHERAPIESYSTEMEN
SURVEILLANCE ET COMMANDE D'ACTIONNEMENT DE DISPOSITIF DANS DES SYSTÈMES DE TRAITEMENT DE PLAIES

(30) Priority: 27.09.2017 US 201762563889 P; 13.07.2018 GB 201811494
(43) Date of publication of application: 05.08.2020
(60) Divisional application: 26196987.7 / 4 826 728
(73) Proprietor: T J Smith & Nephew Limited, Hull, HU3 2BN (GB)
(72) Inventor: BRANDOLINI, Nicola, Hull HU3 2BN (GB); HARTWELL, Edward, Yerbury, Hull HU3 2BN (GB); QUINTANAR, Felix, Clarence, Hull HU3 2BN (GB); VAN RIJ, Johannes, Dagevos, Hull HU3 2BN (GB)
(74) Representative: Appleyard Lees IP LLP
(86) International application number: PCT/EP2018/075751
(87) International publication number: WO 2019/063462

(56) References cited:
- WO-A1-2017/027850
- WO-A1-2017/146986
- DE-U1- 202016 008 343
- US-A1- 2012 109 034
- US-A1- 2015 290 441
- US-A1- 2016 136 339
- US-B2- 8 444 613

## Description

### CROSS-REFERENCE TO RELATED APPLICATIONS

This application claims the benefit of U.S. Provisional Application No. 62/563,889, filed September 27, 2017, and U.K. Provisional Application No. 1811494.2, filed July 13, 2018.

### BACKGROUND

Embodiments of the present disclosure relate to methods and apparatuses for dressing and treating a wound with negative or reduced pressure therapy or topical negative pressure (TNP) therapy. In particular, but without limitation, embodiments disclosed herein relate to negative pressure therapy devices, methods for controlling the operation of TNP systems, and methods of using TNP systems. Prior art devices are disclosed e.g. in US8444613B2, US2015/290441A1, DE202016008343U1 and US2016/136339A1.

### SUMMARY

The invention is defined by the appended claims. In some embodiments, an apparatus for applying negative pressure to a wound is disclosed. The apparatus can include a housing, a pressure source supported by the housing and configured to couple via a fluid flow path to a wound dressing positioned on a wound and provide negative pressure to the wound, a controller supported by the housing and configured to operate the pressure source to provide negative pressure to the wound, and an output device supported by the housing and configured to provide identification data to an electronic device. The identification data being usable by the electronic device to access a label associated with the housing or one or more components supported by the housing.

The apparatus of the preceding paragraph can include one or more of the following features: The identification data can be usable by the electronic device to access the label from a remote database via a computer network. The output device according to the invention as claimed includes a display that is configured to present the identification data as an optical, machine-readable representation of the identification data. The optical, machine-readable representation of the identification data can include a two-dimensional barcode. The output device can include a transmitter configured to wirelessly transmit the identification data to the electronic device. The electronic device can execute an application that receives the identification data from the output device and transmits a request for the label according to the identification data. The electronic device can execute an application that receives the identification data from the output device, transmits via a computer network a request for the label according to the identification data, presents the label on a display to a user, and enables the user of the electronic device to instruct the controller to operate the pressure source to provide negative pressure to the wound dressing. The electronic device can execute an application that transmits a confirmation or a verification of presentation of the label on the display. The controller can determine a location of the housing and automatically select the identification data from a plurality of identification data according to the location. The controller can inactivate the pressure source until the output device provides the identification data to the electronic device. The electronic device can include a mobile personal computer that communicates via a cellular communications network.

In some embodiments, a method is disclosed for operating a wound therapy system. The method can include: retrieving identification data from a memory device of a wound therapy device; outputting the identification data from the wound therapy device to an electronic device; receiving the identification data with the electronic device; generating a request from the identification data with the electronic device, the request being a request to access a label associated with the wound therapy device; transmitting the request with the electronic device via a computer network to a remote database; receiving the label via the computer network; and outputting the label for presentation to a user of the electronic device.

The method of the preceding paragraph can include one or more of the following features: The method can include transmitting via the computer network a confirmation or a verification of the outputting. The outputting the identification data can include presenting, on a display of the negative pressure wound therapy device, the identification data as an optical, machine-readable representation of the identification data, and the receiving the identification data can include receiving the identification data with an image sensor of the electronic device. The outputting the identification data can include wirelessly transmitting the identification data with a transmitter of the negative pressure wound therapy device, and the receiving the identification data can include receiving the identification data with a receiver of the electronic device. According to the invention as claimed, the outputting the label comprises displaying the label on a display of the electronic device.

In some embodiments, an apparatus for applying negative pressure to a wound is disclosed. The apparatus can include a pressure source configured to couple via a fluid flow path to a wound dressing and provide negative pressure to the wound dressing; and a controller. The controller can receive input data, determine a control value from the input data, and adjust an operation performed by the controller according to the control value so that the operation is performed differently than if the operation is performed not according to the control value.

The apparatus of the preceding paragraph can include one or more of the following features: The controller can transmit a verification or a confirmation adjustment to the operation to a remote device via a computer network. The control value can be indicative of operation of the pressure source at an altitude above a threshold, and the operation can be wound therapy performed using the pressure source. The fluid flow path can include a plurality of lumens. The sensor can monitor the pressure at the wound dressing, in one or lumens of the fluid flow path, or at or near an inlet of the pressure source. The controller can activate and deactivate the pressure source.

In some embodiments, an apparatus for applying pressure to a wound is disclosed. The apparatus can include a housing, a motion sensor supported by the housing and configured to output motion data indicative of a motion of the housing, a pressure source supported by the housing, and a controller. The pressure source can couple via a fluid flow path to a wound dressing positioned on a wound and provide negative pressure to the wound. The controller can detect an error condition associated with providing of negative pressure to the wound with the pressure source, determine a cause of the error condition from the motion data, and output an alert for presentation to a user notifying the user of the cause of the error condition.

The apparatus of the preceding paragraph can include one or more of the following features: The error condition can include a blockage in the fluid flow path or a low pressure level at the wound. The controller can determine the blockage from a flow in the fluid flow path or a level of activity of the pressure source. The apparatus can include a canister supported by the housing that may collect fluid aspirated from the wound, and the cause of the error condition can be a rotation of the housing that likely saturated a filter of the canister with the fluid or a vibration of the housing that likely saturated a filter of the canister with the fluid. The controller can output a user instruction to the user indicating how to remedy the cause of the error condition. The user instruction can indicate to replace a filter of a canister, and the canister can be supported by the housing and collect fluid aspirated from the wound. The controller can output a user instruction to the user indicating how to prevent future occurrences of the error condition. The user instruction can indicate not to rotate the housing as detected from the motion. The controller can, responsive to detection of the error condition, operate the pressure source differently than prior to detection of the error condition. The motion sensor can include an accelerometer. The controller can add an entry to a log indicating an occurrence of the error condition, determine a frequency of occurrence of the error condition from the log, and operate the pressure source. The apparatus can include a display, and the display can visually present the alert to the user. The apparatus can include a speaker, and the speaker can audibly present the alert to the user.

In some embodiments, a method for operating a wound therapy device is disclosed. The method can include: operating a pressure source of the wound therapy device to provide negative pressure via a fluid flow path to a wound dressing positioned on a wound, the pressure source being supported by a housing of the wound therapy device; generating motion data indicative of a motion of the housing; detecting an error condition associated with providing of negative pressure to the wound with the pressure source; determining a cause of the error condition from the motion data; and outputting an alert for presentation to a user notifying the user of the cause of the error condition.

The method of the preceding paragraph can include one or more of the following features: The wound therapy device can be a negative pressure wound therapy device. The wound therapy device can include a canister supported by the housing that may collect fluid aspirated from the wound, and the cause of the error condition can be a rotation of the housing that likely caused a filter of the canister to become saturated with the fluid. The method can include outputting a user instruction to the user indicating how to remedy the cause of the error condition. The user instruction can indicate to replace a filter of a canister, and the canister can be supported by the housing and collect fluid aspirated from the wound. The method can include outputting a user instruction to the user indicating how to prevent future occurrences of the error condition or operating the pressure source differently responsive to determining the cause of the error condition.

### BRIEF DESCRIPTION OF THE DRAWINGS

Embodiments of the present disclosure will now be described hereinafter, by way of example only, with reference to the accompanying drawings in which:
Figure 1 illustrates a negative pressure wound therapy system according to some embodiments.
Figures 2A, 2B, and 2C illustrate a TNP apparatus according to some embodiments.
Figures 3A, 3B, 3C, 3D, 3E, 3F, and 3G illustrate components of a negative pressure therapy system according to some embodiments.
Figure 4 illustrates components of a negative pressure therapy system that includes multiple wound dressings according to some embodiments.
Figures 5 and 6 illustrate communications within negative pressure wound therapy systems according to some embodiments.
Figure 7 illustrates a label access process according to some embodiments.
Figures 8A, 8B, 8C, and 8D illustrate user interfaces for managing a label access process and performing other operations according to some embodiments.
Figures 9A and 9B illustrate user interfaces for accessing intellectual property related information according to some embodiments.
Figure 10 illustrates a control process according to some embodiments.
Figure 11 illustrates a monitoring process according to some embodiments.
Figure 12 illustrates a TNP apparatus according to some embodiments.
Figures 13A-13J illustrate plots of motion data over time collected when the TNP apparatus of Figure 12 was moved.

### DETAILED DESCRIPTION

The present disclosure relates to methods and apparatuses for dressing and treating a wound with reduced pressure therapy or topical negative pressure (TNP) therapy. In particular, but without limitation, embodiments of this disclosure relate to negative pressure therapy apparatuses, methods for controlling the operation of TNP systems, and methods of using TNP systems. The methods and apparatuses can incorporate or implement any combination of the features described below. Moreover, the features of this disclosure can be incorporated or implemented in other wound therapy apparatuses, such as positive pressure therapy devices, or other medical apparatuses usable for treating a patient.

TNP therapy can assist in the closure and healing of many forms of "hard to heal" wounds by reducing tissue oedema, encouraging blood flow and granular tissue formation, or removing excess exudate and can reduce bacterial load (and thus infection risk). In addition, TNP therapy may allow for less disturbance of a wound leading to more rapid healing. TNP systems can also assist in the healing of surgically closed wounds by removing fluid or help to stabilize the tissue in the apposed position of closure. A further beneficial use of TNP therapy can be found in grafts and flaps where removal of excess fluid is important and close proximity of the graft to tissue is required in order to ensure tissue viability.

As is used herein, reduced or negative pressure levels, such as -X mmHg, represent pressure levels that are below atmospheric pressure, which typically corresponds to 760 mmHg (or 1 atm, 29.93 inHg, 101.325 kPa, 14.696 psi, etc.). Accordingly, a negative pressure value of -X mmHg reflects pressure that is X mmHg below atmospheric pressure, such as a pressure of (760-X) mmHg. In addition, negative pressure that is "less" or "smaller" than -X mmHg corresponds to pressure that is closer to atmospheric pressure (e.g., -40 mmHg is less than -60 mmHg). Negative pressure that is "more" or "greater" than -X mmHg corresponds to pressure that is further from atmospheric pressure (e.g., -80 mmHg is more than -60 mmHg).

### Overview

In some instances, updates to labelling for a TNP apparatus may be desirable or needed. For example, a label can be updated when an indication of use for the TNP apparatus may be added or changed or when a change may occur in the standards that the TNP apparatus is expected or required to meet (for instance, IEC 60601-1, FCC, etc., as well as if the device is released or operated in a new country with particular compliance rules).

It may be difficult, in some instances, to ensure that a patient is reliably provided up-to-date labelling for a TNP apparatus. For example, the ability of a TNP apparatus to receive and present an updated label may be limited or not dependable. Some features disclosed herein address this technical difficulty by facilitating a TNP apparatus to direct another electronic device to quickly access and present an up-to-date label and thereby relieving the TNP apparatus from the burden of having to provide the up-to-date label. Advantageously, in certain embodiments, these features can reduce the memory or hardware requirements for a TNP apparatus because the TNP apparatus can enable access to the up-to-date label but not itself receive or present the up-to-date label. Moreover, this may increase the robustness and security of a TNP apparatus because the TNP apparatus may not be susceptible to receiving malicious or improper code via an update to label information for the TNP apparatus.

Moreover, some features disclosed herein relate to approaches for making a TNP apparatus more responsive and intelligent when handling various conditions or environments.

### Wound Therapy System

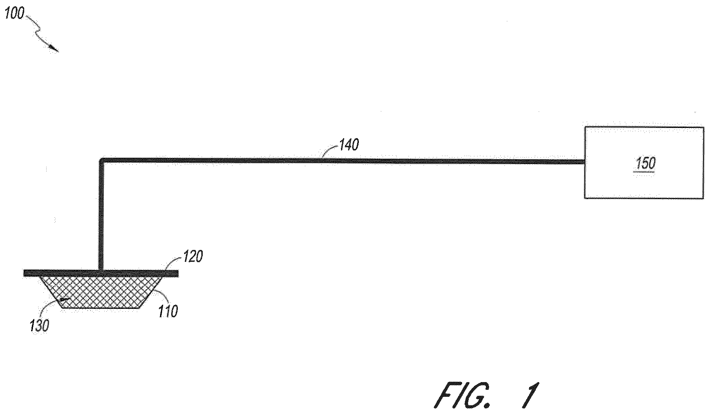

Figure 1 illustrates an embodiment of a negative or reduced pressure wound treatment (or TNP) system 100 comprising a wound filler 130 placed inside a wound cavity 110, the wound cavity sealed by a wound cover 120. The wound filler 130 in combination with the wound cover 120 can be referred to as wound dressing. A single or multi lumen tube or conduit 140 is connected to the wound cover 120 with a TNP apparatus 150 configured to supply reduced pressure. The wound cover 120 can be in fluidic communication with the wound cavity 110. In any of the system embodiments disclosed herein, as in the embodiment illustrated in Figure 1, the TNP apparatus can be a canisterless TNP apparatus (meaning that exudate is collected in the wound dressing or is transferred via tube 140 for collection to another location). However, any of the TNP apparatus embodiments disclosed herein can be configured to include or support a canister. Additionally, in any of the system embodiments disclosed herein, any of the TNP apparatus embodiments can be mounted to or supported by the dressing, or adjacent to the dressing.

The wound filler 130 can be any suitable type, such as hydrophilic or hydrophobic foam, gauze, inflatable bag, and so on. The wound filler 130 can be conformable to the wound cavity 110 such that it substantially fills the cavity. The wound cover 120 can provide a substantially fluid impermeable seal over the wound cavity 110. The wound cover 120 can have a top side and a bottom side, and the bottom side adhesively (or in any other suitable manner) seals with wound cavity 110. The conduit 140 or lumen or any other conduit or lumen disclosed herein can be formed from polyurethane, PVC, nylon, polyethylene, silicone, or any other suitable material.

Some embodiments of the wound cover 120 can have a port (not shown) configured to receive an end of the conduit 140. For example, the port can be Renasys Soft Port available from Smith & Nephew. In other embodiments, the conduit 140 can otherwise pass through or under the wound cover 120 to supply reduced pressure to the wound cavity 110 so as to maintain a desired level of reduced pressure in the wound cavity. The conduit 140 can be any suitable article configured to provide at least a substantially sealed fluid flow pathway between the TNP apparatus 150 and the wound cover 120, so as to supply the reduced pressure provided by the TNP apparatus 150 to wound cavity 110.

The wound cover 120 and the wound filler 130 can be provided as a single article or an integrated single unit. In some embodiments, no wound filler is provided and the wound cover by itself may be considered the wound dressing. The wound dressing may then be connected, via the conduit 140, to a source of negative pressure, such as the TNP apparatus 150. The TNP apparatus 150 can be miniaturized and portable, although larger conventional pumps such can also be used.

The wound cover 120 can be located over a wound site to be treated. The wound cover 120 can form a substantially sealed cavity or enclosure over the wound site. In some embodiments, the wound cover 120 can be configured to have a film having a high water vapor permeability to enable the evaporation of surplus fluid, and can have a superabsorbing material contained therein to safely absorb wound exudate. It will be appreciated that throughout this specification reference is made to a wound. In this sense it is to be understood that the term wound is to be broadly construed and encompasses open and closed wounds in which skin is torn, cut or punctured or where trauma causes a contusion, or any other surficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, acute wounds, chronic wounds, surgical incisions and other incisions, subacute and dehisced wounds, traumatic wounds, flaps and skin grafts, lacerations, abrasions, contusions, burns, diabetic ulcers, pressure ulcers, stoma, surgical wounds, trauma and venous ulcers or the like. The components of the TNP system described herein can be particularly suited for incisional wounds that exude a small amount of wound exudate.

Some embodiments of the system are designed to operate without the use of an exudate canister. Some embodiments can be configured to support an exudate canister. In some embodiments, configuring the TNP apparatus 150 and tubing 140 so that the tubing 140 can be quickly and easily removed from the TNP apparatus 150 can facilitate or improve the process of dressing or pump changes, if necessary. Any of the pump embodiments disclosed herein can be configured to have any suitable connection between the tubing and the pump.

The TNP apparatus 150 can be configured to deliver negative pressure of approximately -80 mmHg, or between about -20 mmHg and 200 mmHg in some implementations. Note that these pressures are relative to normal ambient atmospheric pressure thus, -200 mmHg would be about 560 mmHg in practical terms. The pressure range can be between about -40 mmHg and -150 mmHg. Alternatively a pressure range of up to -75 mmHg, up to -80 mmHg or over -80 mmHg can be used. Also a pressure range of below -75 mmHg can be used. Alternatively a pressure range of over approximately -100 mmHg, or even 150 mmHg, can be supplied by the TNP apparatus 150.

In operation, the wound filler 130 is inserted into the wound cavity 110 and wound cover 120 is placed so as to seal the wound cavity 110. The TNP apparatus 150 provides a source of a negative pressure to the wound cover 120, which is transmitted to the wound cavity 110 via the wound filler 130. Fluid (e.g., wound exudate) is drawn through the conduit 140, and can be stored in a canister. In some embodiments, fluid is absorbed by the wound filler 130 or one or more absorbent layers (not shown).

Wound dressings that may be utilized with the TNP apparatus and other embodiments of the present application include Renasys-F, Renasys-G, Renasys AB, and Pico Dressings available from Smith & Nephew. Further description of such wound dressings and other components of a negative pressure wound therapy system that may be used with the TNP apparatus and other embodiments of the present application are found in U.S. Patent Publication Nos. 2011/0213287, 2011/0282309, 2012/0116334, 2012/0136325, and 2013/0110058, which are incorporated by reference in their entirety. In other embodiments, other suitable wound dressings can be utilized.

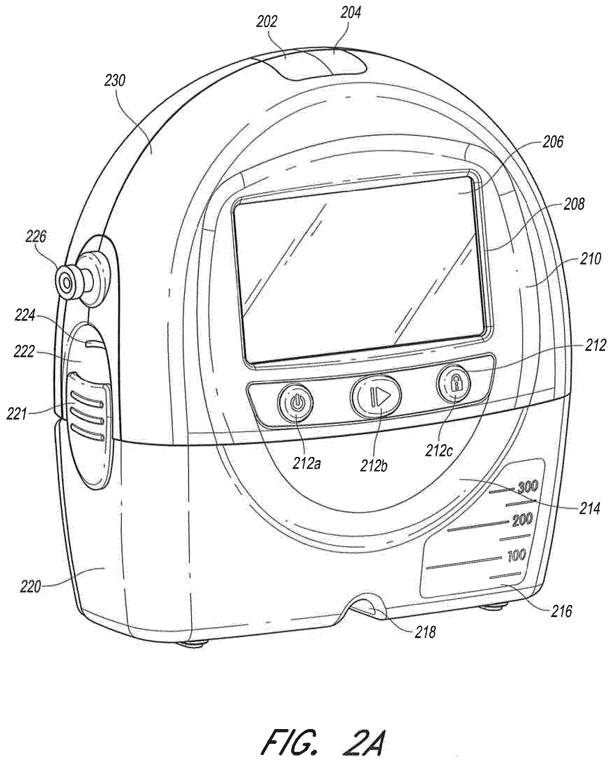

Figure 2A illustrates a front view of a pump assembly 230 and canister 220 according to some embodiments. As is illustrated, the pump assembly 230 and the canister 220 are connected, thereby forming a TNP apparatus. The pump assembly 230 can be similar to or the same as the TNP apparatus 150 in some embodiments.

The pump assembly 230 includes one or more indicators, such as visual indicator 202 configured to indicate alarms and visual indicator 204 configured to indicate status of the TNP system. The indicators 202 and 204 can be configured to alert a user, such as patient or medical care provider, to a variety of operating or failure conditions of the system, including alerting the user to normal or proper operating conditions, pump failure, power supplied to the pump or power failure, detection of a leak within the wound cover or flow pathway, suction blockage, or any other similar or suitable conditions or combinations thereof. The pump assembly 230 can comprise additional indicators. The pump assembly can use a single indicator or multiple indicators. Any suitable indicator can be used such as visual, audio, tactile indicator, and so on. The indicator 202 can be configured to signal alarm conditions, such as canister full, power low, conduit 140 disconnected, seal broken in the wound seal 120, and so on. The indicator 202 can be configured to display red flashing light to draw user's attention. The indicator 204 can be configured to signal status of the TNP system, such as therapy delivery is ok, leak detected, and so on. The indicator 204 can be configured to display one or more different colors of light, such as green, yellow, etc. For example, green light can be emitted when the TNP system is operating properly and yellow light can be emitted to indicate a warning.

The pump assembly 230 includes a display or screen 206 mounted in a recess 208 formed in a case of the pump assembly. The display 206 can be a touch screen display. The display 206 can support playback of audiovisual (AV) content, such as instructional videos. As explained below, the display 206 can be configured to render a number of screens or graphical user interfaces (GUIs) for configuring, controlling, and monitoring the operation of the TNP system. The pump assembly 230 comprises a gripping portion 210 formed in the case of the pump assembly. The gripping portion 210 can be configured to assist the user to hold the pump assembly 230, such as during removal of the canister 220. The canister 220 can be replaced with another canister, such as when the canister 220 has been filled with fluid.

The pump assembly 230 includes one or more keys or buttons configured to allow the user to operate and monitor the operation of the TNP system. As is illustrated, there buttons 212a, 212b, and 212c (collectively referred to as buttons 212) are included. Button 212a can be configured as a power button to turn on/off the pump assembly 230. Button 212b can be configured as a play/pause button for the delivery of negative pressure therapy. For example, pressing the button 212b can cause therapy to start, and pressing the button 212b afterward can cause therapy to pause or end. Button 212c can be configured to lock the display 206 or the buttons 212. For instance, button 212c can be pressed so that the user does not unintentionally alter the delivery of the therapy. Button 212c can be depressed to unlock the controls. In other embodiments, additional buttons can be used or one or more of the illustrated buttons 212a, 212b, or 212c can be omitted. Multiple key presses or sequences of key presses can be used to operate the pump assembly 230.

The pump assembly 230 includes one or more latch recesses 222 formed in the cover. In the illustrated embodiment, two latch recesses 222 can be formed on the sides of the pump assembly 230. The latch recesses 222 can be configured to allow attachment and detachment of the canister 220 using one or more canister latches 221. The pump assembly 230 comprises an air outlet 224 for allowing air removed from the wound cavity 110 to escape. Air entering the pump assembly can be passed through one or more suitable filters, such as antibacterial filters. This can maintain reusability of the pump assembly. The pump assembly 230 includes one or more strap mounts 226 for connecting a carry strap to the pump assembly 230 or for attaching a cradle. In the illustrated embodiment, two strap mounts 226 can be formed on the sides of the pump assembly 230. In some embodiments, various of these features are omitted or various additional features are added to the pump assembly 230.

The canister 220 is configured to hold fluid (e.g., exudate) removed from the wound cavity 110. The canister 220 includes one or more latches 221 for attaching the canister to the pump assembly 230. In the illustrated embodiment, the canister 220 comprises two latches 221 on the sides of the canister. The exterior of the canister 220 can formed from frosted plastic so that the canister is substantially opaque and the contents of the canister and substantially hidden from plain view. The canister 220 comprises a gripping portion 214 formed in a case of the canister. The gripping portion 214 can be configured to allow the user to hold the pump assembly 220, such as during removal of the canister from the apparatus 230. The canister 220 includes a substantially transparent window 216, which can also include graduations of volume. For example, the illustrated 300 mL canister 220 includes graduations of 50 mL, 100 mL, 150 mL, 200 mL, 250 mL, and 300 mL. Other embodiments of the canister can hold different volume of fluid and can include different graduation scale. For example, the canister can be an 800 mL canister. The canister 220 comprises a tubing channel 218 for connecting to the conduit 140. In some embodiments, various of these features, such as the gripping portion 214, are omitted or various additional features are added to the canister 220. Any of the disclosed canisters may include or may omit a solidifier.

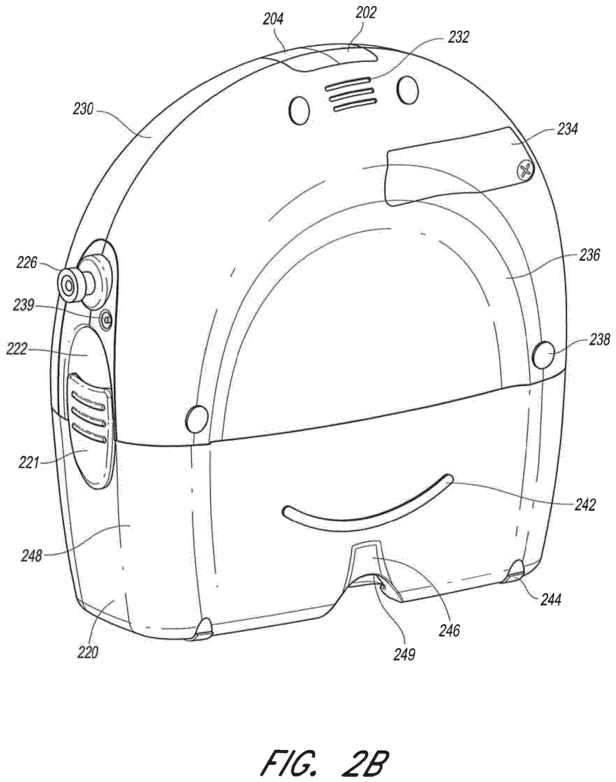

Figure 2B illustrates a rear view of the pump assembly 230 and canister 220 according to some embodiments. The pump assembly 230 comprises a speaker port 232 for producing sound. The pump assembly 230 includes a filter access door 234 with a screw for removing the access door 234, accessing, and replacing one or more filters, such as antibacterial or odor filters. The pump assembly 230 comprises a gripping portion 236 formed in the case of the pump assembly. The gripping portion 236 can be configured to allow the user to hold the pump assembly 230, such as during removal of the canister 220. The pump assembly 230 includes one or more covers 238 configured to as screw covers or feet or protectors for placing the pump assembly 230 on a surface. The covers 230 can be formed out of rubber, silicone, or any other suitable material. The pump assembly 230 comprises a power jack 239 for charging and recharging an internal battery of the pump assembly. The power jack 239 can be a direct current (DC) jack. In some embodiments, the pump assembly can comprise a disposable power source, such as batteries, so that no power jack is needed.

The canister 220 includes one or more feet 244 for placing the canister on a surface. The feet 244 can be formed out of rubber, silicone, or any other suitable material and can be angled at a suitable angle so that the canister 220 remains stable when placed on the surface. The canister 220 comprises a tube mount relief 246 configured to allow one or more tubes to exit to the front of the device. The canister 220 includes a stand or kickstand 248 for supporting the canister when it is placed on a surface. As explained below, the kickstand 248 can pivot between an opened and closed position. In closed position, the kickstand 248 can be latched to the canister 220. In some embodiments, the kickstand 248 can be made out of opaque material, such as plastic. In other embodiments, the kickstand 248 can be made out of transparent material. The kickstand 248 includes a gripping portion 242 formed in the kickstand. The gripping portion 242 can be configured to allow the user to place the kickstand 248 in the closed position. The kickstand 248 comprises a hole 249 to allow the user to place the kickstand in the open position. The hole 249 can be sized to allow the user to extend the kickstand using a finger.

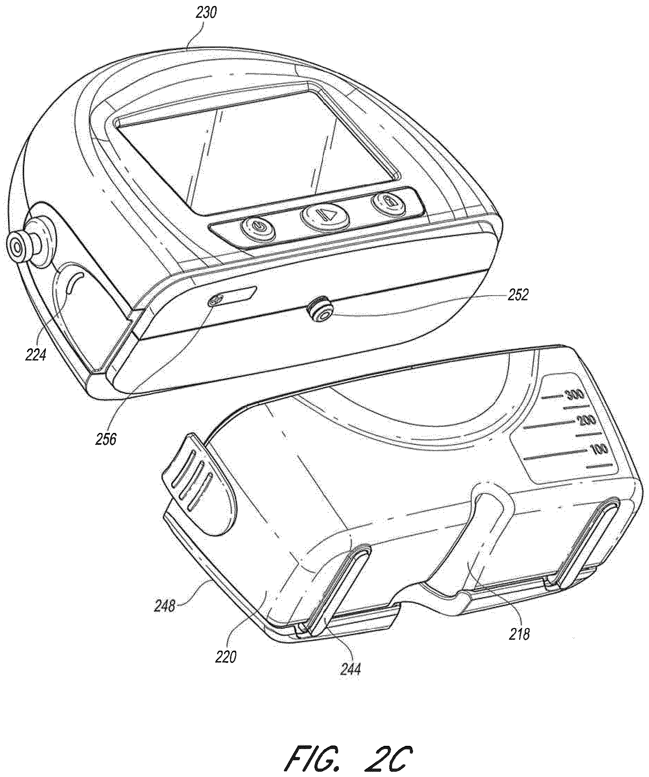

Figure 2C illustrates a view of the pump assembly 230 separated from the canister 220 according to some embodiments. The pump assembly 230 includes a vacuum attachment, connector, or inlet 252 through which a vacuum pump communicates negative pressure to the canister 220. The pump assembly aspirates fluid, such as gas, from the wound via the inlet 252. The pump assembly 230 comprises a USB access door 256 configured to allow access to one or more USB ports. In some embodiments, the USB access door is omitted and USB ports are accessed through the door 234. The pump assembly 230 can include additional access doors configured to allow access to additional serial, parallel, or hybrid data transfer interfaces, such as SD, Compact Disc (CD), DVD, FireWire, Thunderbolt, PCI Express, and the like. In other embodiments, one or more of these additional ports are accessed through the door 234.

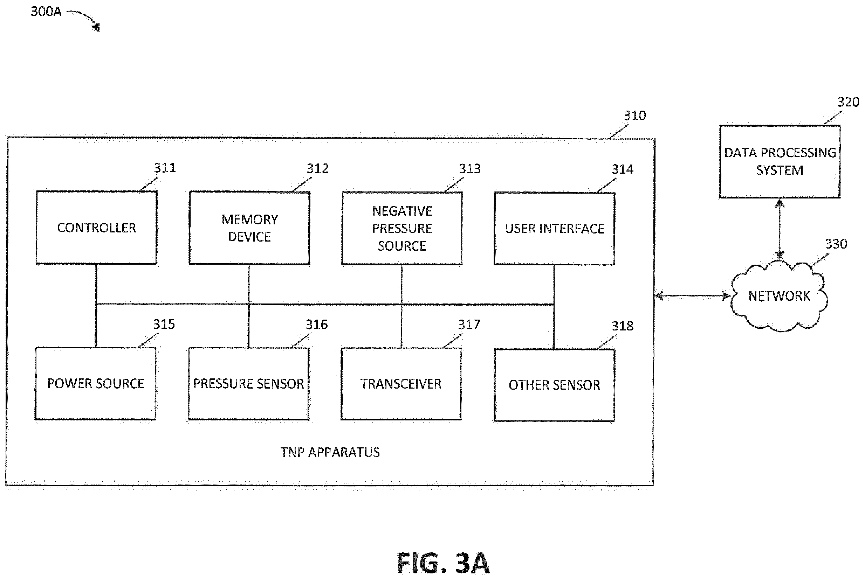

Figure 3A illustrates components of a negative pressure therapy system 300A that includes a TNP apparatus 310 and a remote data processing system 320 according to some embodiments. The TNP apparatus 310 can be used to treat a wound using a wound dressing that is in fluidic communication with the TNP apparatus 310 via a fluid flow path. The TNP apparatus 310 can include a controller 311, a memory device 312, a negative pressure source 313, a user interface 314, a power source 315, a pressure sensor 316, a transceiver 317, and one or more other sensors 318 that are configured to electrically communicate with one another. The power source 315 can provide power to one or more components of the TNP apparatus 310. The TNP apparatus 310 can operate at the pressure levels and using control approaches as described herein or similar to those described in U.S. Patent Publication Nos. 2016/0136339 and 2016/0184496, which are incorporated by reference in their entirety. The TNP apparatus 310 can be similar to or the same as the TNP apparatus 150 in some embodiments.

The controller 311 can control operations of one or more other components of the TNP apparatus 310 according at least to instructions stored in the memory device 312. The controller 311 can, for instance, control operations of and supply of negative pressure by the negative pressure source 313. The negative pressure source 313 can include a pump, such as, without limitation, a rotary diaphragm pump or other diaphragm pump, a piezoelectric pump, a peristaltic pump, a piston pump, a rotary vane pump, a liquid ring pump, a scroll pump, a diaphragm pump operated by a piezoelectric transducer, or any other suitable pump or micropump or any combinations of the foregoing.

The user interface 314 can include one or more elements that receive user inputs or provide user outputs to a patient or caregiver. The one or more elements that receive user inputs can include buttons, switches, dials, touch screens, or the like. The user interface 314 can, for example, be used to generate and display a report or other information reflecting data from therapy use, data from non-compliant use, or a comparison of data from therapy use versus non-compliant use. As another example, the user interface 314 may receive a user input providing a patient reference number or another unique identifier, and the TNP apparatus 310 may then be activated for use by the patient and data collected and stored as described herein may be associated with the patient reference number for usage monitoring for a particular patient. The user interface 314 can also provide an alert to the user. For example, the user interface 314 can include a screen that may visibly present the alert or a speaker that may audibly present the alert.

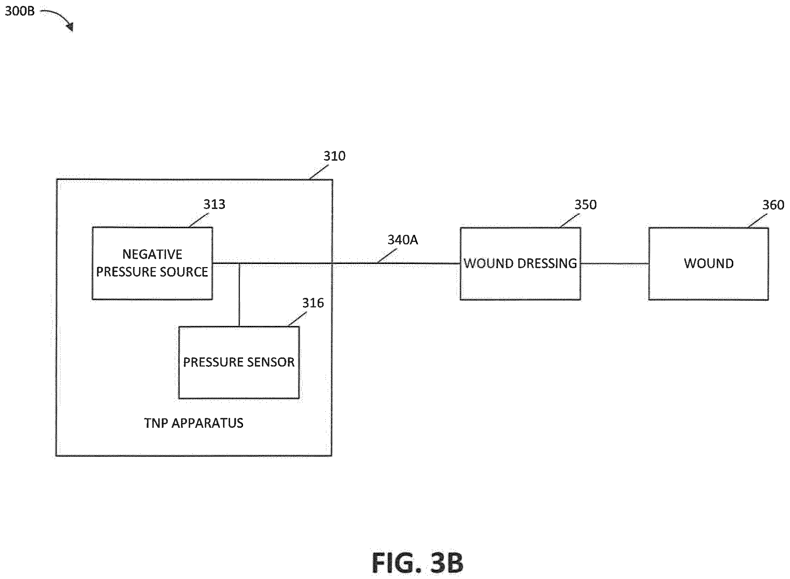

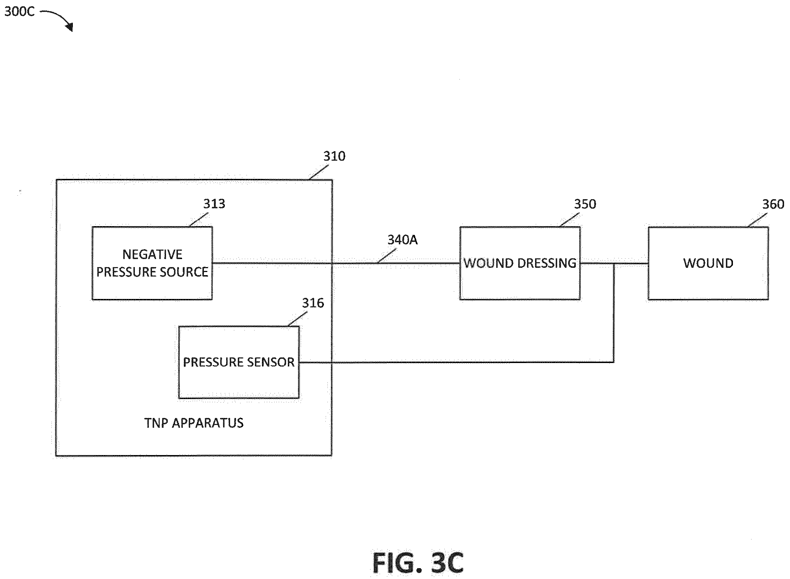

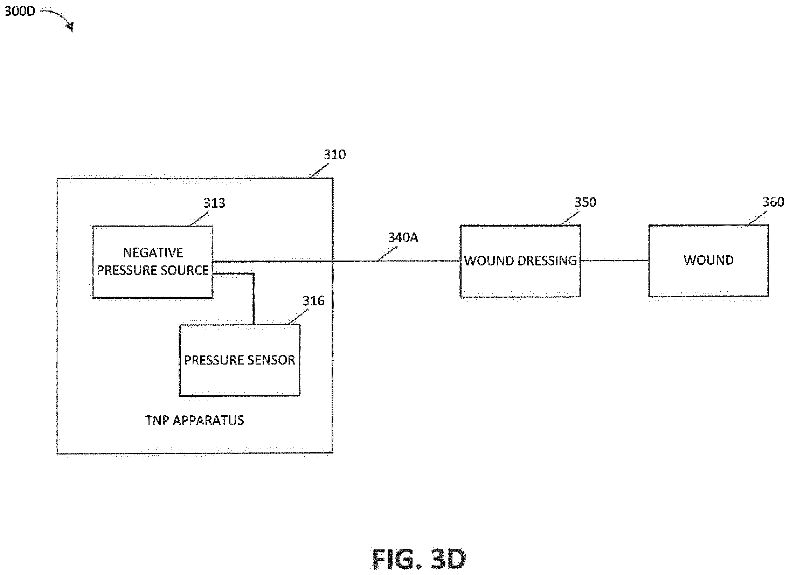

The pressure sensor 316 can be used to monitor pressure underneath a wound dressing, such as (i) pressure in a fluid flow path connecting the negative pressure source 313 and the wound dressing as illustrated by Figure 3B, (ii) pressure at the wound dressing as illustrated by Figure 3C, or (iii) pressure at or in the negative pressure source 313 as illustrated by Figure 3D. As the negative pressure source 313 provides negative pressure, the negative pressure source 313 may generate pressure pulses that are propagated through the fluid flow path and detected by the pressure sensor 316. These pressure pulses may show as a change or bounce in the magnitude or frequency of a signal from the pressure sensor 316.

The controller 311 can analyze a signal output by the pressure sensor 316 to determine pressure in the fluid flow path. The controller 311 may examine the signal using one or more approaches including time domain or frequency domain calculations, such as with a digital signal processor.

The controller 311 or other circuitry of the TNP apparatus 310 may process one or more signals output by the pressure sensor 316 by filtering out noise and then dynamically amplifying the filtered one or more signals. Dynamic amplification can be performed without filtering. This may enable the features described herein to be applied to smaller wounds or weaker pressure signals. For example, the amplification can be performed by a programmable gain amplifier, which may be controlled by software or hardware.

The detection of pressure by the pressure sensor 316 can, in some instances, be enhanced by changing one or more settings of the negative pressure source 313, such as increasing or decreasing vacuum level delivered by the negative pressure source 313, stopping the negative pressure source 313, changing an operating speed of the negative pressure source 313, changing a cadence of the negative pressure source 313, combinations of the same, or the like. The controller 311 can, for example, automatically manage adjustment of the one or more settings.

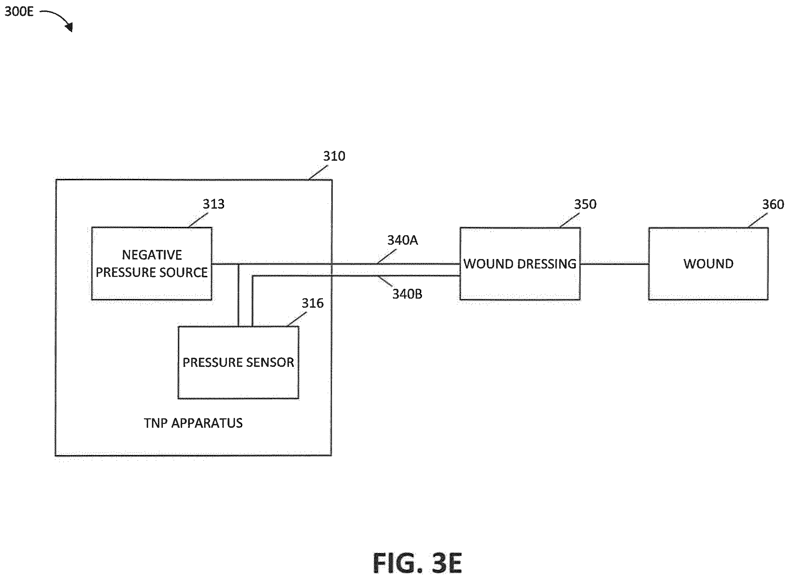

In some implementations, the pressure sensor 316 can be used in combination with another pressure sensor so that the at least two pressure sensors that are positioned in or fluidically connected to the fluid flow path to permit differential measurement of the pressure, such as illustrated by Figure 3E. For example, a first pressure sensor can be positioned upstream of the wound (such as at or near the inlet of the negative pressure source 313C) and a second pressure sensor can be positioned to detect pressure at or near the wound or at or near a canister. This configuration can be accomplished by incorporating, in addition to one or more lumens forming a first fluid flow path connecting the negative pressure source 313 to the wound, a second fluid flow path that includes one or more lumens connecting the TNP apparatus 310 to the wound and through which the second pressure sensor can monitor pressure at or near the wound or at or near a canister. The first and second fluid flow paths can be fluidically isolated from each other. When the at least two pressure sensors are used, the rate of change of pressure (for example, in peak-to-peak pressure or maximum pressure) in the first and second fluid flow paths can be determined and the difference in pressure detected between the first and second pressure sensors can be determined. These values can be used separately or together to detect various operational conditions, such as leaks, blockages, canister full, presence of blood in the first fluid flow path or the second fluid flow path, etc. In some implementations, multiple redundant pressure sensors can be provided to protect against failure of one or more of the pressure sensors.

The transceiver 317 can be used to communicate with the data processing system 320 via a network 330. The transceiver 317 can, for example, transmit device usage data like alarms, measured pressure, or changes to a therapy program administered by the TNP apparatus 310 to the data processing system 320. The network 330 can be a communication network, such as a wireless communications network like a cellular communications network. The memory device 312 can be used to store the device usage data that may be transmitted by the transceiver 317.

The one or more other sensors 318 can be or include one or more motion sensors (for example, an accelerometer, gyroscope, inertial measurement unit, or orientation detector). The TNP apparatus 310 can adjust its operation according to one or more outputs from the one or more other sensors 318. The one or more other sensors 318 can be attached to an outside of a housing of the TNP apparatus 310. The one or more other sensors 318 can be removable, such that it can be interchangeably replaced with another type sensor. Additionally or alternatively, the one or more other sensors 318 can be positioned or placed within a housing of the TNP apparatus 310.

The one or more other sensors 318 can detect multiple parameters, such as acceleration in x, y, z direction or an angle between the orientation of the TNP apparatus 310 and the direction of gravity. Further, by taking measurement multiple times within an interval, the one or more other sensors 318 may provide information about a change in acceleration in x, y, z direction or change in angle between the orientation of the TNP apparatus 310 and the direction of gravity. In some implementations, the one or more other sensors 318 can take measurement 50 times per second, more than 50 times per second, more than 100 times per second, or more than 200 time per second. If data is taken more frequently, a change in acceleration or orientation may be calculated more accurately. The one or more other sensors 318 can communicate sensor data wirelessly, such as via Bluetooth^{®} protocol, to the controller 311 or other components of the TNP apparatus 310.

The output from the one or more other sensors 318 can provide information about various conditions of or situations around the TNP apparatus 310 such that the TNP apparatus 310 may operate depending on such conditions or situations. For example, from the output of the one or more other sensors 318, the TNP apparatus 310 can be determined to be oriented upside-down, shocked, used by a user that may be walking, or positioned within a vehicle or an airplane.

The controller 311 can analyze a signal output by the one or more sensors 318, such as the one or more motion sensors, to determine a motion or orientation of the device as described herein.

The data processing system 320 can, in some implementations, analyze pressure data received from the transceiver 317 to determine whether the received pressure data is indicative of the negative pressure source 313 being in use on a patient, such as using analysis approaches as described with respect to the TNP apparatus 310. The data processing system 320 can, for instance, generate and display a report or other information reflecting data from therapy use, data from non-compliant use, or a comparison of data from therapy use versus non-compliant use. In one example, a user of the data processing system 320 may input a patient reference number or TNP apparatus number associated with a TNP apparatus, and the data processing system 320 can then provide or display data like data from therapy use or data from non-compliant use for the patient reference number or TNP apparatus number.

Figure 3B illustrates a negative pressure therapy system 300B that includes the TNP apparatus 310 of Figure 3A, as well as a first fluid flow path 340A, a wound dressing 350, and a wound 360 according to some embodiments. The TNP apparatus 310 can be used to treat the wound 360 using the wound dressing 350 that is in fluidic communication with the negative pressure source 313 via the first fluid flow path 340A. In particular, Figure 3B depicts that the pressure sensor 316 can be positioned in the first fluid flow path 340A, such as at or near an inlet of the TNP apparatus 310, to measure pressure in the first fluid flow path 340A.

Figure 3C illustrates a negative pressure therapy system 300C that differs from the negative pressure therapy system 300B in that the pressure sensor 316 can instead be positioned to measure pressure at or near the wound dressing 350, such as pressure underneath the wound dressing 350 when the wound dressing 350 is coupled to the wound 360.

Figure 3D illustrates a negative pressure therapy system 300D that differs from the negative pressure therapy system 300B in that the pressure sensor 316 can instead be positioned to measure pressure at the negative pressure source 313. In one example, the pressure sensor 316 can be a part of and within the negative pressure source 313 to measure pressure generated by the negative pressure source 313. In another example, the pressure sensor 316 can be separate from the negative pressure source 313 and positioned to measure pressure at or near an inlet of the negative pressure source 313.

Figure 3E illustrates a negative pressure therapy system 300E that differs from the negative pressure therapy system 300B in that the negative pressure therapy system 300E further includes a second fluid flow path 340B, and the pressure sensor 316 can be a differential pressure sensor or include two pressure sensors. If the pressure sensor 316 may include the two pressure sensors, one of the two pressure sensors of the pressure sensor 316 can be positioned in the first fluid flow path 340A to measure pressure in the first fluid flow path 340A, and the other of the two pressure sensors the pressure sensor 316 can be positioned in the second fluid flow path 340B to measure pressure in the second fluid flow path 340B. If the pressure sensor 316 may be the differential pressure sensor, the pressure sensor 316 can be fluidically connected to the first fluid flow path 340A and the second fluid flow path 340B. The first fluid flow path 340A can thus be used by the negative pressure source 313 to provide negative pressure to the wound dressing 350, and the second fluid flow path 340B can be used primarily by the pressure sensor 316 to measure pressure at or near the wound dressing 350, such as under the wound dressing 360. The pressure sensor 316 can thereby be used by the TNP apparatus 310 to perform differential measurement of pressure between pressure supplied by the negative pressure source 313 and pressure at or near the wound dressing 350.

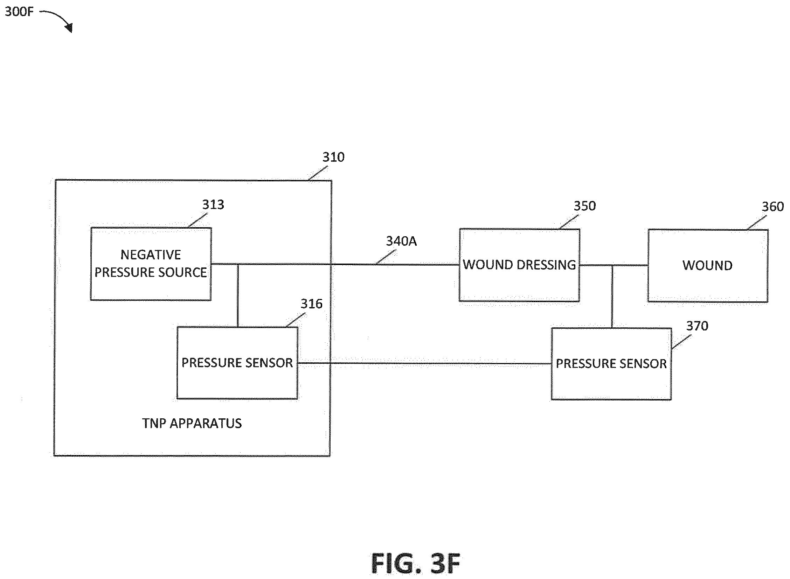

Figure 3F illustrates a negative pressure therapy system 300F that differs from the negative pressure therapy system 300B in that the negative pressure therapy system 300F can further include an additional pressure sensor 370 positioned to measure pressure at or near the wound dressing 350, such as pressure underneath the wound dressing 350 when the wound dressing 350 is coupled to the wound 360. The additional pressure sensor 370 can generate and output a signal to the TNP apparatus 310 responsive to the pressure measured at the wound dressing 350. The pressure sensor 316 and the additional pressure sensor 370 can thus be used by the TNP apparatus 310 to perform differential measurement of pressure between pressure supplied by the negative pressure source 313 and pressure at or near the wound dressing 350.

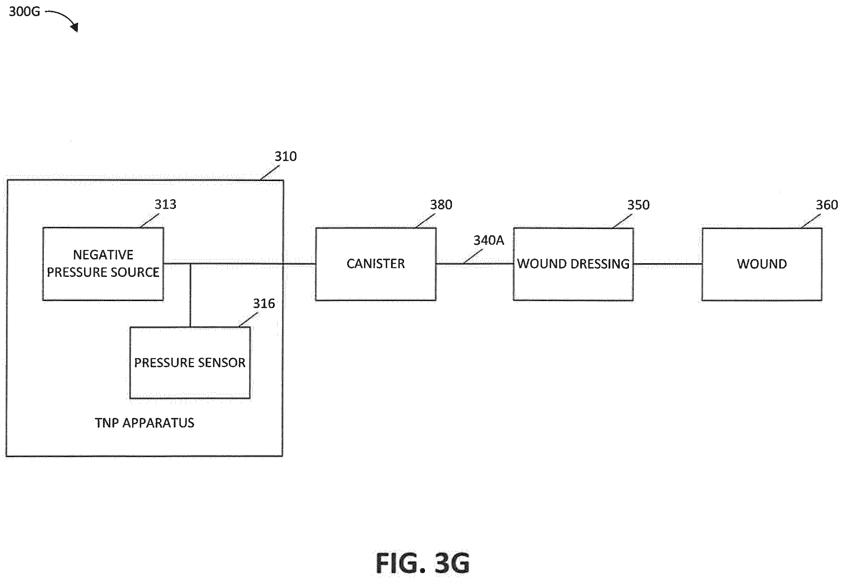

Figure 3G illustrates a negative pressure therapy system 300G that differs from the negative pressure therapy system 300B in that a canister 380 can be coupled between the negative pressure source 313 and the wound dressing 350 in the first fluid flow path 340A. The canister 380 can collect exudate removed from the wound 360. The examples of Figures 3C-3F can be similarly modified to also include the canister 380, in some implementations.

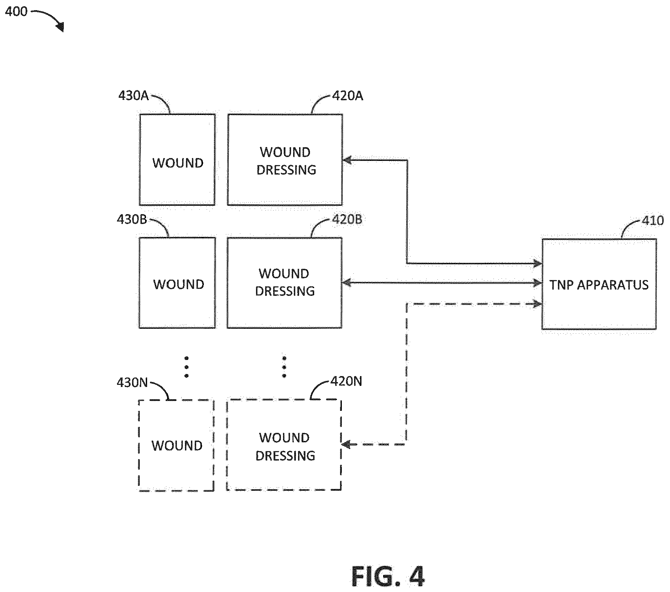

Figure 4 illustrates a negative pressure therapy system 400 that includes a TNP apparatus 410 and wound dressings 420A, 420B, ..., 420N according to some embodiments. The wound dressings 420A, 420B, ..., 420N can be in fluidic communication with the TNP apparatus 410 and each be used to treat a different wound of wounds 430A, 430B, ..., 430N on a patient. The TNP apparatus 410 can be similar to or the same as the TNP apparatus 310 in some embodiments.

The TNP apparatus 310 can separately monitor each of the wound dressings 420A, 420B, ..., 420N so that the TNP apparatus 310 is able to generate an alarm for a subset of the wounds 430A, 430B, ..., 430N (for instance, one, two, or three of the wounds) without having to generate an alarm for the one or more other of the wounds 430A, 430B, ..., 430N. As a result, control can be consolidated with the TNP apparatus 310 and multiple TNP apparatuses may not be used for treating the wounds 430A, 430B, ..., 430N.

In yet further implementations, the negative pressure therapy system 400 can include two of the TNP apparatus 410 where the two of the TNP apparatus 410 communicate with one another to facilitate treatment of more wounds. The negative pressure therapy system 400 in some such implementations may also include a central hub device (not shown) that operates one or both of the TNP apparatus 410 and provides a communication interface for the two of the TNP apparatus 410 through which the two of the TNP apparatus 410 communicate.

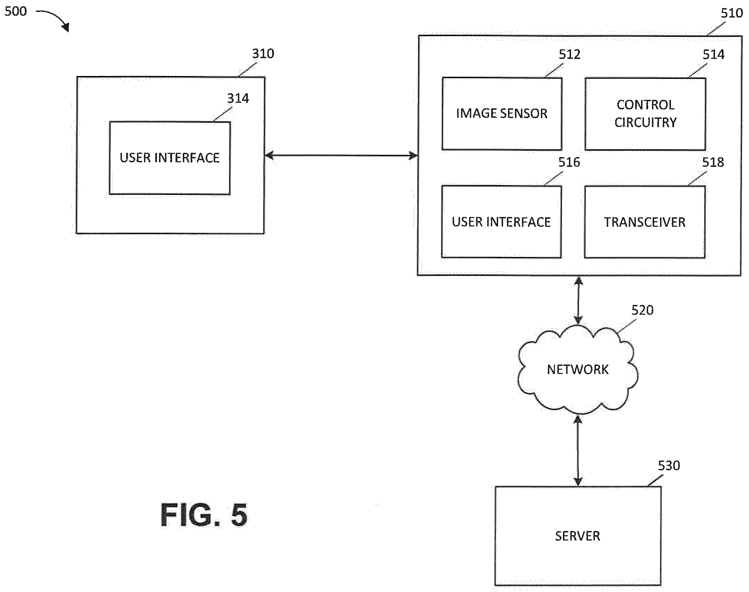

Figure 5 illustrates communications within a negative pressure wound therapy system 500 according to some embodiments. The negative pressure wound therapy system 500 includes the TNP apparatus 310 of Figure 3, as well as an electronic device 510 and a server 530. The TNP apparatus 310 can communicate with the electronic device 510 wirelessly, such as via electromagnetic radiation like optical radiation (for example, light visible to a person). The electronic device 510 can, in turn, wirelessly communicate with the server 530 via a network 520, such as a computer network. The electronic device 510 can, for example, be a smart phone, tablet, personal computer, or the like.

The electronic device 510 can include an image sensor 512, control circuitry 514, a user interface 516, and a transceiver 518. The image sensor 512 can be configured to detect optical radiation, such as in the form of a barcode, displayed by the user interface 314. The control circuitry 514 can process the optical radiation from the user interface 314 and determine to communicate with the server 530 via the network 520 using the transceiver 518. The user interface 316 can be used to display information, such as a label for the TNP apparatus 310, received from the server 530. Moreover, operations of the electronic device 510 and the TNP apparatus 310 can be controlled by a user via the user interface 516.

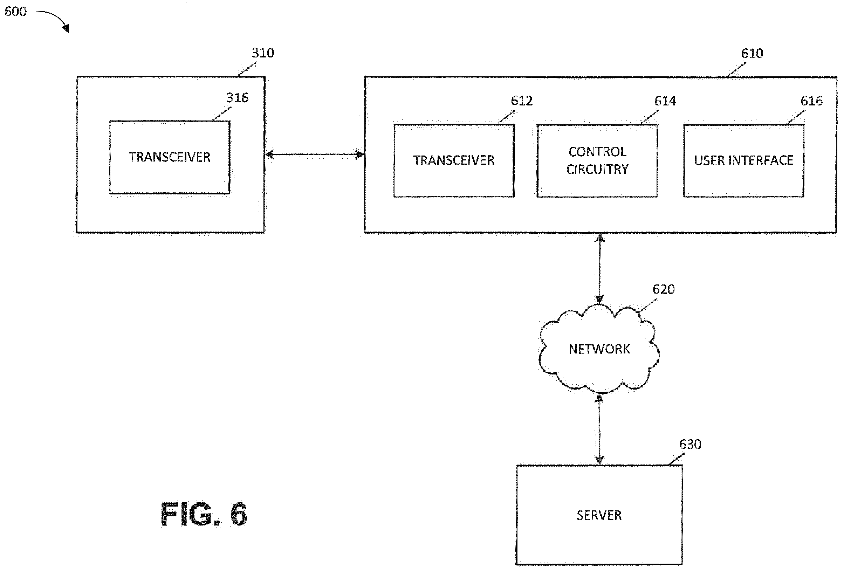

Figure 6 illustrates communications within a negative pressure wound therapy system 600 according to some embodiments. The negative pressure wound therapy system 600 includes the TNP apparatus 310 of Figure 3, as well as an electronic device 610 and a server 630. The negative pressure wound therapy system 600 can be similar to the negative pressure wound therapy system 500; however, the TNP apparatus 310 and the electronic device 610 can be configured to communicate via the transceiver 316 and a transceiver 612 of the electronic device 610 using ultra high frequency (UHF) radiation (for example, around 2.4 GHz radiation) or super high frequency (SHF) radiation (for example, around 5 GHz radiation) rather than optical radiation. The transceiver 316 and the transceiver 612 can, for instance, communicate via data packets and using a communication protocol, such as Bluetooth. The electronic device 610, the network 620, and the server 630 may otherwise operate similarly respectively to the electronic device 510, the network 520, and the server 530, and the transceiver 612 can communicate via the network 620 with the server 530. Control circuitry 614 of the electronic device 610 can be used to display information, such as a label for the TNP apparatus 310, received from the server 630, and operations of the electronic device 610 and the TNP apparatus 310 can be controlled by a user via the user interface 616.

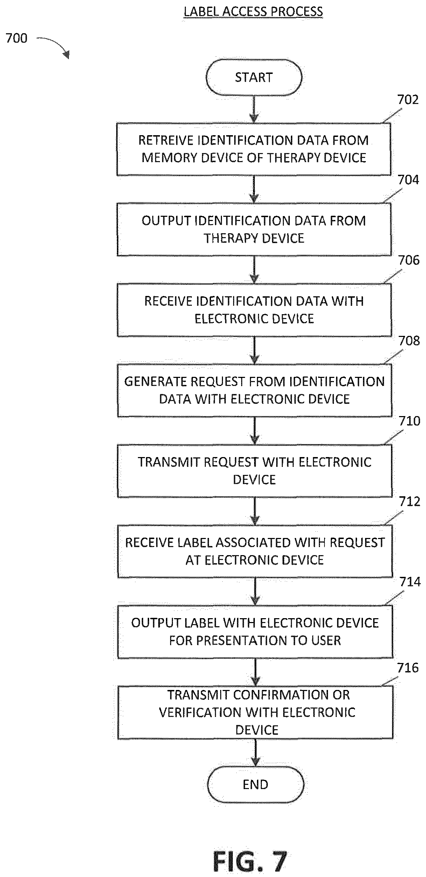

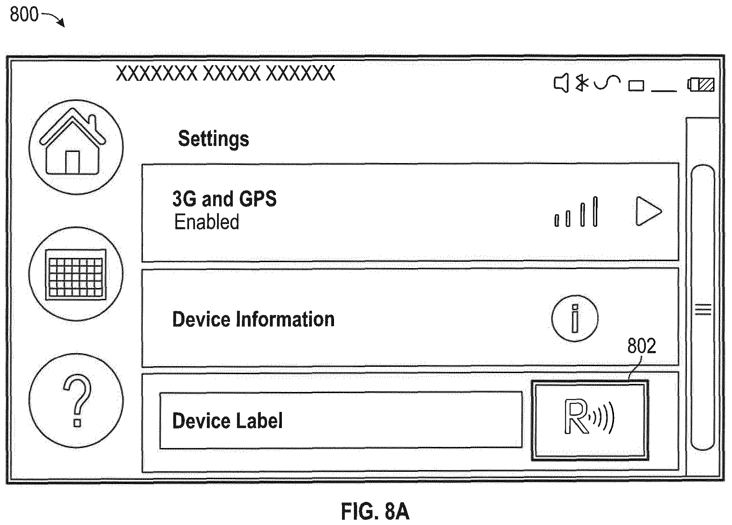

Figure 7 illustrates a label access process 700 according to some embodiments. The label access process 700 can be performed by a therapy system, such as the negative pressure wound therapy system 500 or the negative pressure wound therapy system 600. The label access process 700 can be initiated, for instance, when a user selects a device label area 802 on a menu screen 800 as shown in Figure 8A and that is displayed on a user interface of a TNP apparatus, such as the TNP apparatus 310.

For convenience, the label access process 700 is described in the context of the negative pressure wound therapy system 500 and the negative pressure wound therapy system 600, but may instead be implemented in other systems described herein or by other systems not shown. Advantageously, the label access process 700 provides, in certain embodiments, an approach for a TNP apparatus to provide an up-to-date label without the TNP apparatus itself receiving or presenting updated label information.

At block 702, the label access process 700 can retrieve identification data from a memory device of a therapy device. For example, the TNP apparatus 310 can retrieve identification data from the memory device 312. The identification data can be data usable to access an up-to-date label for the TNP apparatus 310.

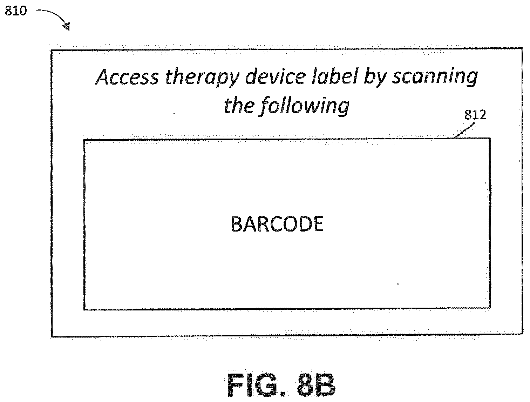

At block 704, the label access process 700 can output the identification data from the therapy device. For example, the user interface 314 can output the identification data by presenting the identification data as a two-dimensional barcode on a display of the user interface 314. The two-dimensional barcode may, for instance, be displayed in a barcode area 812 of a label access screen 810 as shown in Figure 8B and that is displayed on a user interface of a TNP apparatus, such as the TNP apparatus 310. As another example, the user interface 314 can output the identification data by outputting the identification data wirelessly via the transceiver 316. In some implementations, the two-dimensional barcode or the identification data can include information such as an identifier associated with the TNP apparatus 310 or its type of apparatus, as well as an address or other directions for contacting the server 530 to request the up-to-date label.

At block 706, the label access process 700 can receive the identification data output by the therapy device with an electronic device. For example, the electronic device 510 can detect the two-dimensional barcode output by the user interface 314 with the image sensor 512. As another example, the electronic device 610 can detect the identification data output wirelessly by the transceiver 316 with the transceiver 612.

At block 708, the label access process 700 can generate a request from the identification data with the electronic device. For example, the control circuitry 516 can generate a request from the two-dimensional barcode to access to the up-to-date label for the TNP apparatus 310. As another example, the control circuitry 614 can generate a request from the wirelessly received identification data to access to the up-to-date label for the TNP apparatus 310.

At block 710, the label access process 700 can transmit the request with the electronic device. For example, the transceiver 518 can transmit the request via the network 520 to the server 530, or the transceiver 612 can transmit the request via the network 620 to the server 630. The request can be usable by the server 530 or the server 630 to retrieve and provide the up-to-date label for the TNP apparatus 310 to the electronic device 510 or the electronic device 610.

At block 712, the label access process 700 can receive the label associated with the request at the electronic device. For example, the electronic device 510 can receive the up-to-date label via the network from the server 530, or the electronic device 610 can receive the up-to-date label via the network from the server 630. The server 530 may have provided the up-to-date label to the electronic device 510 in response to the request from the electronic device 510, and the server 630 may have provided the up-to-date label to the electronic device 610 in response to the request from the electronic device 610.

The label can include information such as intended purpose of the therapy device, general therapy device warnings, related therapy device supplies and materials, therapy device components, conditions of therapy device use, user preparation information, regulatory numbers or codes for the therapy device or components thereof, name and place of business of manufacturer or distributor, unique therapy device identifiers, combinations of the same, or the like for the TNP apparatus 310. The label can include information in the form of symbols, pictures, alphanumeric characters, combinations of the same, or the like. Some or all of the information of the label can be encrypted prior to communication to the electronic device in view of the potentially safety critical nature of the information and to prevent, for instance, tampering with of the information by others. In some embodiments, the electronic device or the therapy device can hold one or more encryption keys to which message headers point for decryption.

At block 714, the label access process 700 can output the label with the electronic device for presentation to a user. For example, the electronic device 510 can present the label to a user on a display of the user interface 514, or the electronic device 610 can present the label to a user on a display of the user interface 616.

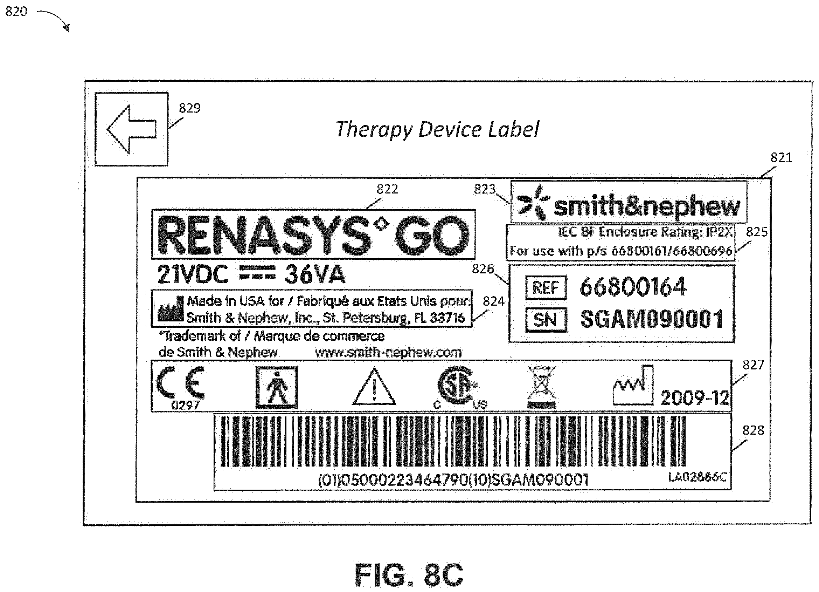

The label can, for instance, be displayed in a label area 821 of a label display screen 820 as shown in Figure 8C and that is displayed on a user interface of an electronic device, such as the electronic device 510 or the electronic device 610. The label as displayed can include a therapy device name section 822 presenting a therapy device name, a therapy device provider name section 823 presenting a therapy device provider name, a provider contact information section 824 presenting provider contact information, a usage information section 825 presenting usage information, a regulatory or serial number section 826 presenting regulatory or serial numbers for the therapy device, a symbols section 827 presenting symbols denoting features of or related to the therapy device, and a machine-readable code section 828 presenting a barcode or the like. In some implementations, the label displayed in the label area 821 can include one or more other sections not shown, a subset of the sections shown in Figure 8C, or multiple repeat or similar sections to those shown in Figure 8C.

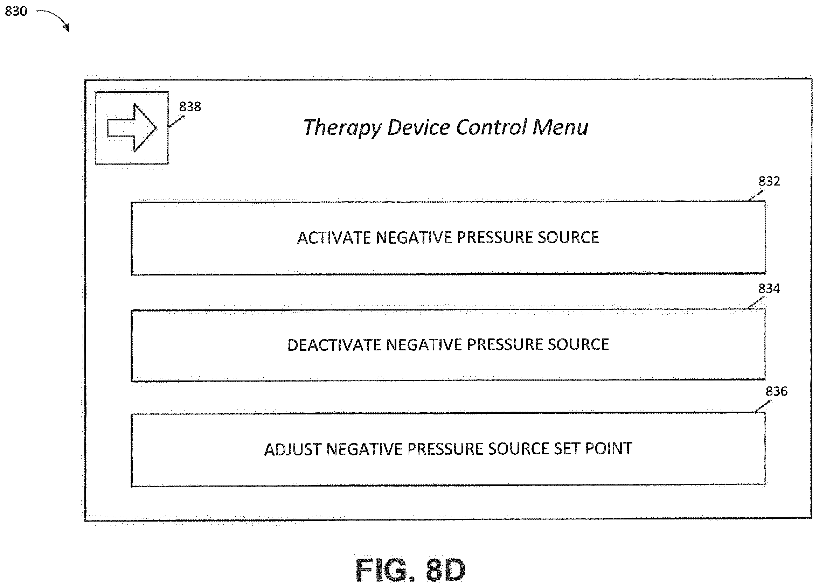

The label display screen 820 can also include a navigation control section 829 that, upon selection by a user, can cause navigate within an application running on an electronic device to another screen, such as a control screen 830 as shown in Figure 8D. The control screen 830 can include an activation area 832 selectable by a user to activate a therapy device like the TNP apparatus 310, a deactivation area 834 selectable by the user to deactivate the therapy device, and an adjustment area 836 selectable by the user to adjust settings of the therapy device like a pressure set point. The control screen 830 can also include a navigation control section 838 that, upon selection by a user, can cause navigate within an application running on an electronic device to another screen, such as the label display screen 820.

At block 716, the label access process 700 can transmit a confirmation or verification with the electronic device. For example, the transceiver 518 can transmit the confirmation or verification via the network 520 to the server 530, or the transceiver 612 can transmit the confirmation or verification via the network 620 to the server 630. The confirmation or verification may include a code usable to confirm or verify that some or all of the information of the label has been presented by the display or that a correct or latest version of the label has been presented by the display. Moreover, the confirmation or verification can be indicative of a readiness of the TNP apparatus 310 (such as for use to perform therapy) and usable to monitor a therapy compliance for the TNP apparatus 310 (such as for device manufacturer, prescribing physician, or insurance provider). In one example, the confirmation or verification can include a combination of a device serial number and a software/data file update version, which may be logged in a database for regulatory or post market surveillance and maintenance use. The confirmation or verification can be encrypted or unique to a certain type of devices, which may provide increased cybersecurity protection. The confirmation or verification can be generated in some implementations at least from a label file. In some embodiments, one or more features of the TNP apparatus 310 or the electronic device 510 or the electronic device 610 may be disabled until the confirmation or verification has be generated or transmitted.

The label access process 700 can further include one or more other features in certain implementations. For example, a therapy device may not operate to provide therapy until the identification data is output by the therapy device. In another example, a therapy device may not operate to provide therapy until the identification data is output by the therapy device and the therapy device receives confirmation from an electronic device receiving the identification data that the identification data has been used to present an up-to-date label.

The electronic device or the therapy device can include one or more cybersecurity mechanisms to protect the integrity of their operations. For example, the electronic device or the therapy device may store data received from a remote server (such as, updated label or other information) on a memory which is physically separate from another memory used to control or operate therapy, so that malicious code may not be placed to adjust operations even if the communication with the remote server is compromised. In some instances, a control software of the electronic device or the therapy device can verify the data received from the remote server. When therapy settings are received remotely, the settings may be stored in a memory separate from the control software, such that the control software can check the memory for a setting against any pre-defined protocol or other limit. If the protocol or other limit is not met, the control software may not operate the electronic device or the therapy device.

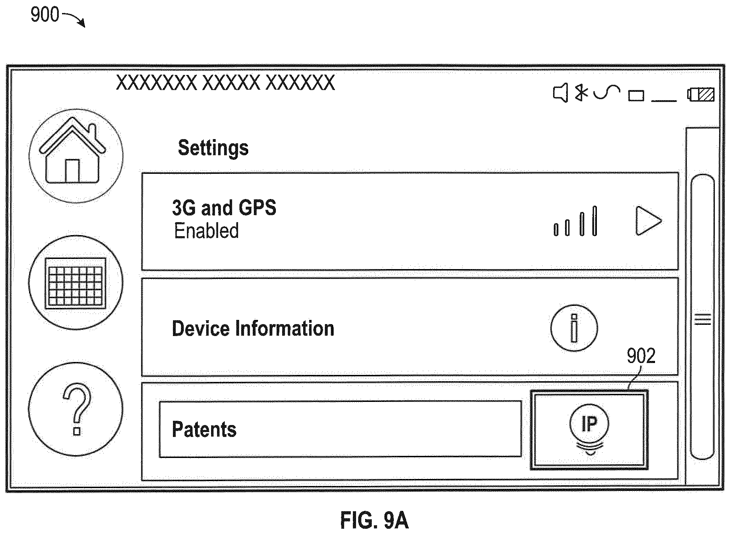

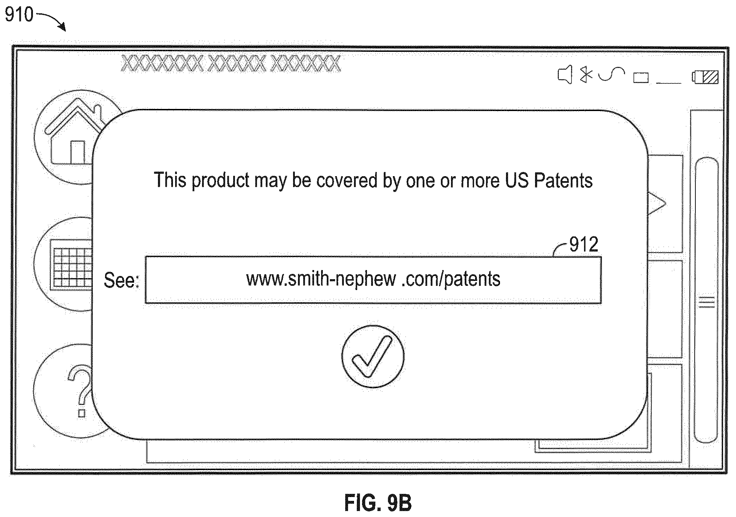

Figures 9A illustrates a menu screen 900 that is displayable on user interface of a therapy device, such as the TNP apparatus 310. The menu screen 900 can include a patents area 902 selectable by a user to view patent or other intellectual property information related to the therapy device. Moreover, upon selection of the patents area 902, a patents screen 910 as shown in Figure 9B can appear in place of the menu screen 900. The patents screen 910 can include a patents link area 912, which may be selectable by a user to load a link, such as a Uniform Resource Locator (URL), in an application like a browser.

### Therapy Device Control Process

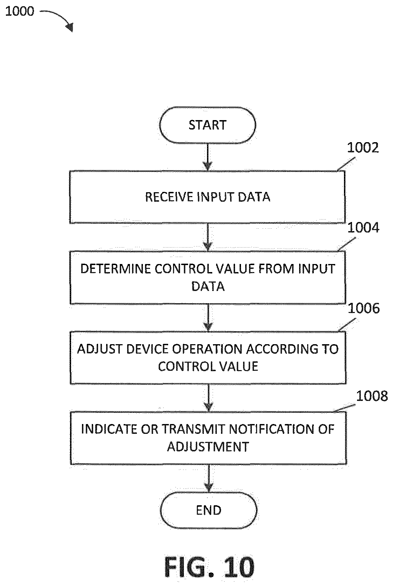

FIG. 10 illustrates a control process 1000 performable by a device, such as the TNP apparatus 150 of Figure 1, the pump assembly 230 of Figure 2A-C, the TNP apparatus 310 of Figure 3A, or other TNP apparatuses like those described in U.S. Patent Publication Nos. 2016/0136339 and 2016/0184496 that were previously incorporated herein by reference in their entireties. For convenience, the control process 1000 is described in the context of the TNP apparatus 310 of Figure 3A, but may instead be implemented in other systems described herein or by other systems not shown.

The control process 1000 can advantageously enable the TNP apparatus 310 to perform more efficiently, effectively, or safely than other TNP apparatuses, such as by functioning dynamically and intelligently to prevent degradation in performance of therapy, prevent device misuse, help ensure that therapy is completed, preserve performance of the negative pressure source 313, or ensure that the negative pressure source 313 adapts to a changing external environment.

At block 1002, the controller 311 can receive input data. The input data can include, for instance, device operating information, information about the environment around the TNP apparatus 310, information about current conditions of a patient using the TNP apparatus 310, or prescribed therapy and information. The input data can be collected using a sensor (such as the pressure sensor 316 or the one or more other sensors 318), a user input (such as via the user interface 314), or a received control input (such as via a communication received by the transceiver 317 from another device via the network 330), among other possible sources like those described herein or the like.

At block 1004, the controller 311 can determine a control value from the input data. The control value can be, for example, a control parameter usable to adjust an operation controlled by the controller 311.

At block 1006, the controller 311 can adjust a device operation according to the control value. The controller 311 can, for instance, change a processing by the TNP apparatus 310 (such as an alarming, an applied pressure control algorithm, when or how to report data, when or how to collect inputs used to alter processing, device power usage, or noise suppression in signals) according to the control value.

At block 1008, the controller 311 can indicate adjustment of the device operation or transmit a notification of adjustment of the device operation. The controller 311 can, for example, set a flag in a memory device indicating successful or unsuccessful adjustment or transmit a confirmation or verification indicative of the successful or unsuccessful adjustment to another device, such as via a communications network.

### Remote Programming and Local Confirmation Example

The control process 1000 can be the process by which remote programming is manually validated locally at the TNP apparatus 310. The data processing system 320 can provide via the network 330 an instruction message to the TNP apparatus 310 to function according to certain instructions, such as a prescribed treatment for a patient assigned to use the TNP apparatus 310. To ensure, however, that the function provided by the certain instructions are appropriate and safe for the patient, a caregiver can use the user interface 314 to review and confirm the function and provide authentication that activates the function. In the absence of the local authentication by an appropriate caregiver, the TNP apparatus 310 can receive the certain instructions but may not function according to the certain instructions. In one implementation, the caregiver may have caused the data processing system 320 to provide the certain instructions, and the local authentication can thus provide a check that the certain instructions were correctly received or to be implemented by the TNP apparatus 310. Once the local authentication has been received, the TNP apparatus 310 can further transmit a confirmation or verification of receipt of the local authentication, such as to another device via a communications network. For instance, the confirmation or verification can be indicative of a readiness of the TNP apparatus 310 (such as for use to perform therapy) and usable to monitor a therapy compliance for the TNP apparatus 310. In one implementation, the confirmation or verification can include a combination of a device serial number and a software/data file update version as described herein that may be encrypted or unique to a certain type of devices. Additionally or alternatively, a failure to successfully perform the local authentication can cause the TNP apparatus 310 to transmit failure notification, such as to another device via a communications network.

The authentication described in the preceding paragraph can be additionally or alternatively implemented using one or more approaches. For example, the authentication can be performed using a validation code entered via the user interface 314, via a radio-frequency identification (RFID) tag, or a handheld device of the caregiver (such as a smart phone). Moreover, the authentication described in the preceding paragraph can be desirable, in certain instances, for use in a home healthcare setting where a clinician in a patient's home may review and confirm the certain instructions.

### Two-Way Control of Negative Pressure Source Example

The control process 1000 can be the process by which the TNP apparatus 310 is remotely set into different modes, such as a home-use mode (for instance, where the pressure settings for using the TNP apparatus 310 may not be changed) or a hospital mode (for instance, where the pressure settings for using the TNP apparatus 310 may be changed). The data processing system 320 can provide via the network 330 an instruction to the TNP apparatus 310. The TNP apparatus 310 can, in turn, adjust its mode according to the instruction. In some implementations, the change in mode by the TNP apparatus 310 can trigger an alarm of the user interface 314 or an alarm at the data processing system 320 via a communication from the TNP apparatus 310 through the network 330.

### Alarm Setting Example

The control process 1000 can be the process by which the TNP apparatus 310 changes its alarming (such as by (i) raising or lowering an alarm sensitivity like an alarm threshold for audibly or visibly alarming depending on a particular mode or (ii) adjust help screen shown to the patient depending on a particular mode) according to whether a patient using the TNP apparatus 310 may be at a particular location, moving around, or situated in a certain environment.

For instance, the TNP apparatus 310 can have an ambulatory mode or a stationary mode. The mode of the TNP apparatus 310 can be set according to a user input to the user interface 314 or via a sensor input using a sensor like a motion sensor (for example, an accelerometer or gyroscope) or an orientation detector. The TNP apparatus 310 can activate an audible or visible alarm of user interface 314 or display a particular help screen depending both device operating parameters and the mode of the device. In another instance, the TNP apparatus 310 can suppress or alternatively present one or more alarms presented by the TNP apparatus 310 when the TNP apparatus 310 determines that its location is within an area (such as, using GPS data or Wi-Fi or location communication triangulation data collected by the TNP apparatus 310) where a caregiver would be expected to be present, such as at a hospital. In yet another instance, the TNP apparatus 310 can suppress or alternatively present one or more alarms presented by the TNP apparatus 310 when the TNP apparatus 310 determines that a local time where the TNP apparatus 310 is positioned (which may be automatically determined by the TNP apparatus 130 with GPS data or Wi-Fi or location communication triangulation data collected by the TNP apparatus 310) falls within a suppression period (such as, during late night hours when a patient would be expected to be sleeping) of an alarm suppression schedule, which may be programmed at manufacture or set or adjusted by user input to the user interface 314.

As another example, the TNP apparatus 310 can determine a transportation environment (for instance, transportation in an automobile, train, or airplane) in which the TNP apparatus 310 is positioned. The transportation environment can be set according to a user input to the user interface 314 or via a sensor output from the one or more other sensors 318 (for instance, a motion sensor or an audio sensor for detect frequencies of vibration or noise). In one example, when the one or more other sensors 318 includes a motion sensor, the output of the motion sensor can be used to detect a movement pattern indicative of a certain transportation or the audio sensor detects certain noise or vibration, such as in a range of 1 Hz to 1KHz, over a threshold level, and the TNP apparatus 310 can, for instance, adjust certain settings like the alarm sensitivity (such as, by decreasing the sensitivity) or sound volume (such as, by increasing the volume). In environments where low noise or vibration levels are detected, the sensitivity of alarms may, for instance, be increased or sound volume may be decreased.

The alarm sensitivity adjustment described in the preceding paragraph can, in some implementations, be used to adjust a threshold for triggering an alarm to indicate a blockage. As described in U.S. Patent Publication Application No. 2016/0184496, the entire disclosure of which is hereby incorporated by reference in its entirety, peak-to-peak measurements of pressure can be used to detect a blockage. In one example, a trigger for the blockage alarm in one condition can be counting the number of peak-to-peak measurements that exceed a threshold level in a period of time. When the sensitivity of alarms is reduced, the peak-to-peak threshold can, for example, be increased for the period of time. As a result, the alarming for a therapy device may be a less sensitive when a patient is walking than when a patient is riding in a moving vehicle.

### Noise Rejection or Suppression Example

The control process 1000 can be the process by which the TNP apparatus 310 adjusts its functioning to reject or suppress noise so that the TNP apparatus 310 may continue to accurately function. The TNP apparatus 310 can, for example, stop operating when the TNP apparatus 310 detects an environment of high interference (such as, a high temperature, humidity, position, or acceleration) or operate more conservatively and with lower confidence that instructed operations are being performed. The TNP apparatus 310 can, moreover, communicate data to the data processing system 320 via the network 330 when the TNP apparatus 310 detects that an interference level around the TNP apparatus 310 or in the network 330 is below an interference threshold.

### Power Management Example

The control process 1000 can be the process by which the TNP apparatus 310 shuts off one or more components or services provided by the TNP apparatus 310 depending on a remaining amount of energy or operating temperature of the power source 315. This can desirably, in certain embodiments, enable the TNP apparatus 310 to preserve power for operating the negative pressure source 313.

### Location Services Selection Example

The control process 1000 can be the process by which the TNP apparatus 310 selects from one or more sources of location information. For instance, in response to the TNP apparatus 310 determining that the patient is moving (such as using a motion sensor or an orientation sensor), the TNP apparatus 310 can attempt to use Wi-Fi or location communication triangulation data to determine the location of the TNP apparatus 310 rather than GPS data. Moreover, in response to the TNP apparatus 310 determining that the patient is stationary (such as using the motion sensor or the orientation sensor), the TNP apparatus 310 can attempt to use GPS data to determine the location of the TNP apparatus 310 rather than Wi-Fi or location communication triangulation data.

### Control Using Analog Rather Than Digital Example

The control process 1000 can be the process by which the TNP apparatus 310 selects to use analog data or digital data to control the negative pressure source 313. For instance, when operating in certain environments like high noise environments (such as, when the therapy device may be exposed to significant amounts of motion, electromagnetic radiation, or heat), an analog pressure sensor may provide a more accurate pressure reading than a digital pressure sensor for use in the controller 311 controlling the negative pressure source 313.

### User Interface Menu Configuration Example

The control process 1000 can be the process by which the TNP apparatus 310 configures behavior of menus of the user interface 314 according to environmental conditions detected by the TNP apparatus 310.

The TNP apparatus 310 can, for example, automatically display a particular help screen on the user interface 314 responsive to detecting a certain detected environmental condition (for instance, using a motion sensor, an orientation sensor, or other sensor) associated with the particular help screen. The particular help screen may present information usable by a user of the TNP apparatus 310 to diagnose and resolve the detected environmental condition. The TNP apparatus 310 can thus timely present the particular help screen on the user interface 314 in anticipation of the user seeking out the particular help screen to address the environmental condition.

In yet another example, the TNP apparatus 310 can simplify one or more user interfaces displayed to a user (for instance, by reducing an amount of presented data, reducing a number of available inputs, or changing a presentation scheme such as to have different colors, interface element sizes, or presented durations of interface elements) when the TNP apparatus 310 detects an environmental condition associated with a less friendly environment for operating the TNP apparatus 310, such as when the TNP apparatus 310 is determined to be moving (such as using a motion sensor or an orientation sensor) or a detected ambient temperature is below a first temperature threshold (for instance, 30°F, 40°F, or 50°F) or is above a second temperature threshold (for instance, 100°F, 110°F, or 120°F).

In yet another example, the TNP apparatus 310 can vary an amount of user interface interactivity that is requested from a user depending on a determined patient health or activity level (such as, may be determined from a user input indicating patient health or activity level or inferred from one or more past user inputs or detected conditions around the TNP apparatus 310 or about the user. For instance, the TNP apparatus can simplify one or more user interfaces displayed to a user (such as, by reducing an amount of presented data, reducing a number of available inputs, or changing a presentation scheme such as to have different colors, interface element sizes, or presented durations of interface elements) when the TNP apparatus 310 detects less than a threshold amount of movement, or when the TNP apparatus 310 receives other information like vital signs that indicate the patient may not healthy enough to provide much user input.

### Flow-Based Pressure Control Example

The control process 1000 can be the process by which the TNP apparatus 310 automatically increases pressure provided by the negative pressure source 313 responsive to determining that a flow of liquid has increased from a wound to which the negative pressure is provided. This increase can desirably, in certain embodiments, help prevent a decrease in an effectiveness of therapy provided by the TNP apparatus 310 as the flow of liquid from the wound increases.

### Time Configuration Example

The control process 1000 can be the process by which the TNP apparatus 310 automatically determines local time, date, or daylight savings data, and accordingly adjusts its settings. The TNP apparatus 310 may include a communications module, such as a 3G module, which enables the TNP apparatus 310 to obtain the local time, date, and daylight savings time data from a computer network like a cellular network. In response to the TNP apparatus 310 determining local time, date or daylight savings data, the TNP apparatus 310 can adjust its display or use of time, date, or any other time or date associated data for the TNP apparatus 310. As a result, a user of the TNP apparatus 310 may or may not need to manually provide time information, so a possibility of use error is reduced. Further, the TNP apparatus 310 may or may not need to have an internal clock which keeps running from user start-up or manufacture of the TNP apparatus 310. In some implementations, the TNP apparatus 310 can use GPS data to obtain a location of the TNP apparatus 310 to automatically determine a local time as described herein.

### Altitude Configuration Example

The control process 1000 can be the process by which the TNP apparatus 310 determines an altitude of where the TNP apparatus 310 is located and adjusts one or more therapy parameters according to the determined altitude. For instance, the TNP apparatus 310 may include a sensor, such as an altimeter, atmospheric pressure sensor, accelerometer, or GPS sensor, usable to detect altitude at which the TNP apparatus 310 is positioned. In response to determining that the detected altitude is within one or more altitude ranges or above a threshold (for example, above 10,000 ft. altitude), the TNP apparatus 310 can adjust one or more therapy parameters, such as a pressure setting level, pressure variation pattern, mode of operation of pressure source, alarm threshold, alarm sensitivity, sensor sensitivity, or the like.

### Hyperbaric Chamber Example

The control process 1000 can be the process by which the TNP apparatus 310 detects the presence of a hyperbaric chamber nearby and adjusts an operation of the TNP apparatus 310 when the hyperbaric chamber is detected. For instance, the TNP apparatus 310 can include one or more sensors to detect an elevated ambient pressure level or an elevated ambient oxygen level, which may be caused by the use of a hyperbaric chamber. In response to the detection of the hyperbaric chamber, the TNP apparatus 310 can adjust its operation (such as, by powering off the TNP apparatus 310, diminishing an operating power level, or deactivating certain functionality) to reduce a risk of fire.

### Motion Detection Example

The control process 1000 can be the process by which the TNP apparatus 310 detects a movement or orientation of the TNP apparatus 310 and adjusts its operation according to the movement or orientation. In some instances, the TNP apparatus 310 may determine an acceleration or orientation of the TNP apparatus 310 using the one or more other sensors 318 where the one or more other sensors 318 includes one or more motion sensors. The TNP apparatus 310 further select an operation mode based at least on the detected acceleration or orientation, and adjust an operation of the controller 311 according to the operation mode.

The control process 1000 may be useful for adjusting the operation of the TNP apparatus 310 in various situations. For example, the TNP apparatus 310 may experience an error condition that may trigger an alarm where the error condition may be caused by an inversion of the TNP apparatus 310 that saturates a filter positioned between the canister and a negative pressure source of the TNP apparatus 310 with fluid stored in the canister. In another instance, when the TNP apparatus 310 may detect an aircraft environment, the TNP apparatus 310 can automatically turn off certain wireless data communication functionality (for instance, 3G-GPS communications) so that a user may not have to manually turn off the functionality as may be required by regulatory authorities. The TNP apparatus 310 can additionally or alternatively change the sound volume of alarm according to the operation mode or change a threshold value for a blockage alarm at least based on an acceleration or orientation of the TNP apparatus 310.

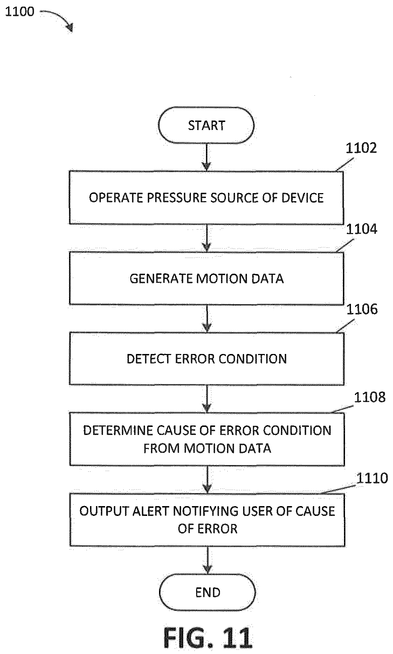

Figure 11 illustrates a monitoring process 1100 according to some embodiments. The monitoring process 1100 can be performed by a therapy system, such as the negative pressure wound therapy system 300A. The therapy system can include a TNP apparatus having a motion sensor, such as the TNP apparatus 310 having the one or more other sensors 318 including the one or more motion sensors. The monitoring process 1100 can be initiated, for instance, when a user selects a motion detecting on/off area (not shown) on a user interface of the TNP apparatus, such as the menu screen 800 as shown in Figure 8A. In some instances, the monitoring process 1100 can be initiated automatically when the TNP apparatus is active or powered.

For convenience, the monitoring process 1100 is described in the context of the negative pressure wound therapy system 300A but may instead be implemented in other systems described herein or by other systems not shown. Advantageously, the monitoring process 1100 provides, in certain embodiments, an approach for a TNP apparatus to determine a cause of an error condition and provide information about the cause of the error condition.

At block 1102, the monitoring process 1100 can operate a pressure source of a therapy device. For example, the TNP apparatus 310 can operate the negative pressure source 313.

At block 1104, the monitoring process 1100 can generate motion data indicative of a motion of the therapy device using a motion sensor. For example, the TNP apparatus 310 can generate motion data indicative of a motion of a housing of the TNP apparatus 310 using the one or more sensors 318. The motion data can include acceleration, direction of acceleration, change in acceleration, or an angle formed with the direction of gravity, among other information. Various motion data and determination of a motion of the device from motion data are further described herein. The motion data can be stored or recorded in a log in the memory device 312 by the TNP apparatus 310 to permit the motion data to be accessed later.

At block 1106, the monitoring process 1100 can detect an error condition associated with providing of negative pressure to the wound with the pressure source. For example, the error condition can be a blockage in the fluid flow path or a low pressure level at the wound. The TNP apparatus 310 may determine the blockage from a flow in the fluid path or a level of activity of the negative pressure source 313 from pressure values determined using the pressure sensor 316. The error condition may or may not trigger an alarm by the TNP apparatus 310. Examples of error conditions, including leaks or blockages, are described in U.S. Patent Publication Nos. 2015/0025482, 2016/0184496, 2017/0216501, which are incorporated by reference in their entirety. The TNP apparatus 310 can additionally create or add to an entry in a log indicating the occurrence of the error condition. The log may be stored in the memory device 312. In some instances, the TNP apparatus 310 can determine a frequency of the error condition from the log. The TNP apparatus 310 can operate the negative pressure source 313 differently than prior to the detection of the error condition. For instance, the TNP apparatus 310 can deactivate the negative pressure source 313 or change a set point or mode of operation for the negative pressure source 313, among other possibilities.

At block 1108, the monitoring process 1100 can determine a cause of the error condition from the motion data. The cause of the error condition may be determined from the motion data generated prior to, at the time of, or subsequent to the occurrence of the error condition. The TNP apparatus 310 can, for example, analyze the motion data for one or more features indicative of particular causes of error conditions or compare the motion data to model motion data indicative of particular causes of error conditions. If the TNP apparatus 310 determines that the motion data, such as over a duration of time (for example, 0.2, 0.5, 1, 1.5, 2, 3, 5, 10, 20, or 30 seconds), satisfies a threshold associated with a feature indicative of a particular cause or has a threshold degree of similarity to the model motion data associated with a certain cause, the TNP apparatus 310 can determine that the particular or certain cause is the cause of the error condition.

In one example, the TNP apparatus 310 may include a canister, such as the canister 220 of Figures 2A-C, which is supported by a housing of the TNP apparatus 310, such that the canister can collect fluid aspirated from the wound. When the housing of the TNP apparatus 310 is mishandled, such as rotated or vibrated inappropriately, it may cause a filter between the canister and a pump assembly of the apparatus to become saturated with the fluid within the canister. The monitoring process 1100 can detect a threshold magnitude of the rotation or vibration from the motion data and determine that a blockage detected by the TNP apparatus 310 may likely be caused by the canister becoming saturated with the fluid due to the the rotation or vibration. As another example, exertion of the unusual shock to the TNP apparatus 310 can be detected from the motion data, and the shock can be determined to be the cause of a blockage or leak. Other examples of improper handling of the TNP apparatus 310 are further described herein.

At block 1110, the monitoring process 1100 can output an alert for presentation to a user notifying the user of the cause of the error condition. The alert may be presented together with or separate from an alarm associated with the error condition. The alert can identify an improper rotation, vibration, shock, or other motion of the housing of the TNP apparatus 310 or may identify how a motion of the housing resulted in an error condition associated with one or more components of the TNP apparatus 310. The alert can be visually or audibly presented to the user. In one example, the TNP apparatus 310 can output a warning or an alarm, such as for presentation via the user interface 314, notifying that the filter of the canister may be saturated due to a rotation or vibration of the housing of the TNP apparatus 310.

The TNP apparatus 310 can additionally or alternatively output a user instruction to the user, such as for presentation via the user interface 314, indicating how to remedy the error condition. For example, the TNP apparatus 310 may output a user instruction to replace the filter of the canister responsive to the detection of the rotation or vibration that likely caused the filter of the canister to become saturated with the fluid. In some instances, the TNP apparatus 310 may output a user instruction to the user indicating how to prevent future occurrences of the error condition, such as how not to repeat the cause of the error condition. For example, the TNP apparatus 310 can indicate not to rotate or vibrate the housing of the TNP apparatus 310 as detected from the motion data, such as by outputting one or more images or videos for presentation to the user that illustrate proper or improper device handling corresponding to the cause of the error condition.

In addition to or instead of outputting an alert or a user instruction, the monitoring process 1100 can operate differently responsive to determining the cause of the error condition, such as by operating the negative pressure source 313 differently than prior to determining the cause of the error condition. For instance, the TNP apparatus 310 can deactivate the negative pressure source 313 or change a set point or mode of operation for the negative pressure source 313, among other possibilities.

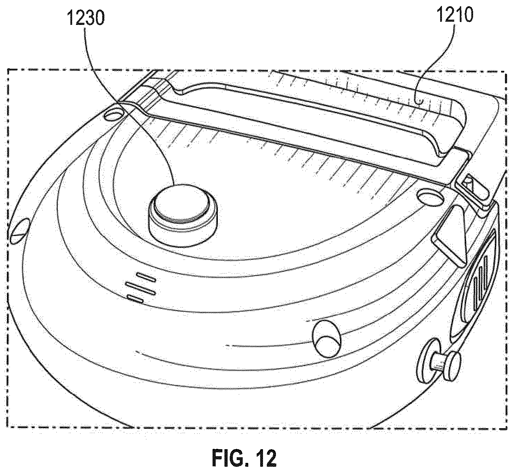

Figure 12 illustrates a TNP apparatus 1210, which can be similar to the pump assembly 230 and the canister 220 of Figure 2A and further include a motion sensor 1230, which can be similar to the one or more other sensors 318 of Figure 3A. The motion sensor 1230 can be attached to a housing of the TNP apparatus 1210. The motion sensor 1230 can detect a movement or an orientation of the TNP apparatus 1210.

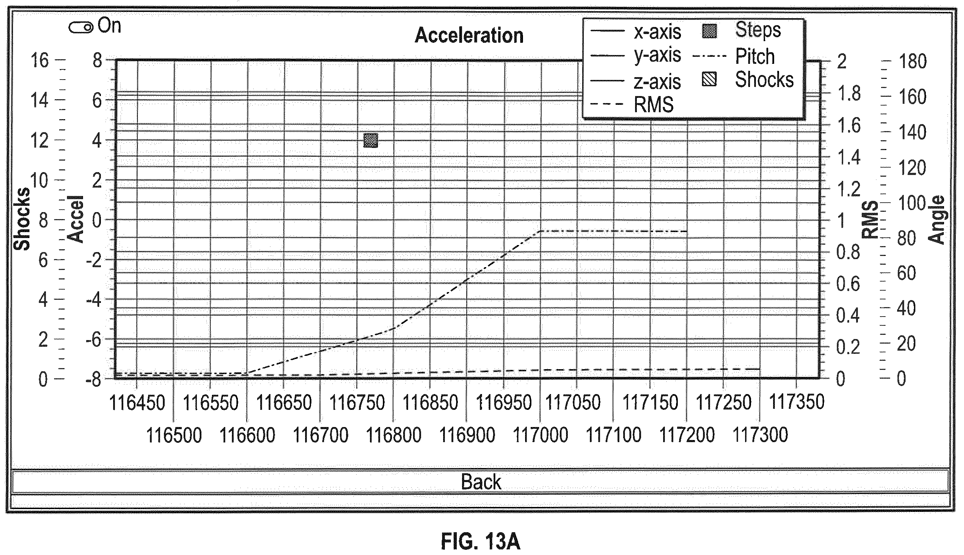

Figures 13A-13J illustrate plots of measurements from a motion sensor attached to a TNP apparatus, such as the motion sensor 1230 of Figure 12 or another motion sensor described herein. In Figures 13A-13J, "Steps" can be determined and output by the motion sensor 1230. "Pitch" may indicate the orientation of the TNP apparatus, and can be determined by the angle formed by the motion sensor 1230 with respect to the direction of gravity. "RMS" can be determined by a root-mean-square of the accelerations measured by the motion sensor 1230 in x, y, and z directions. "Shock" can be determined and output by the motion sensor 1230 and indicative of large changes in acceleration.

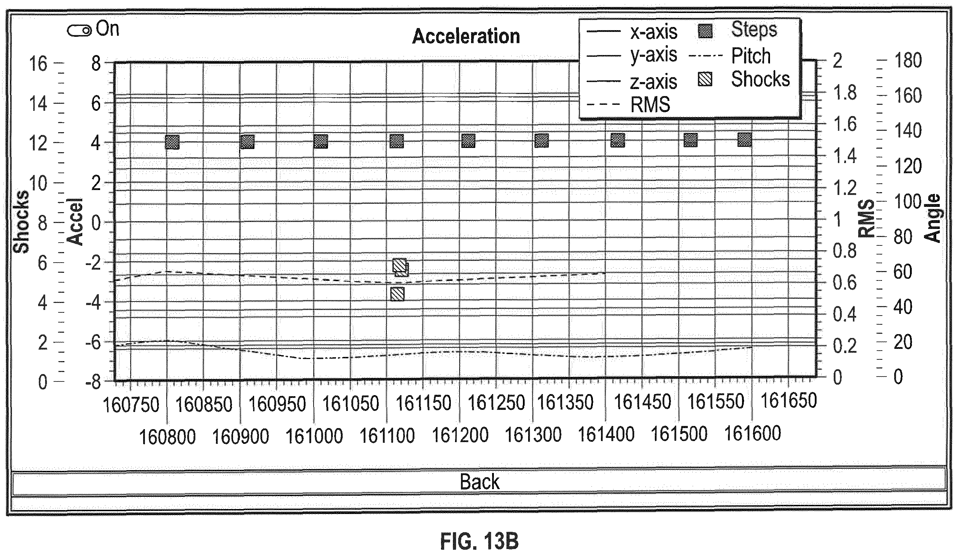

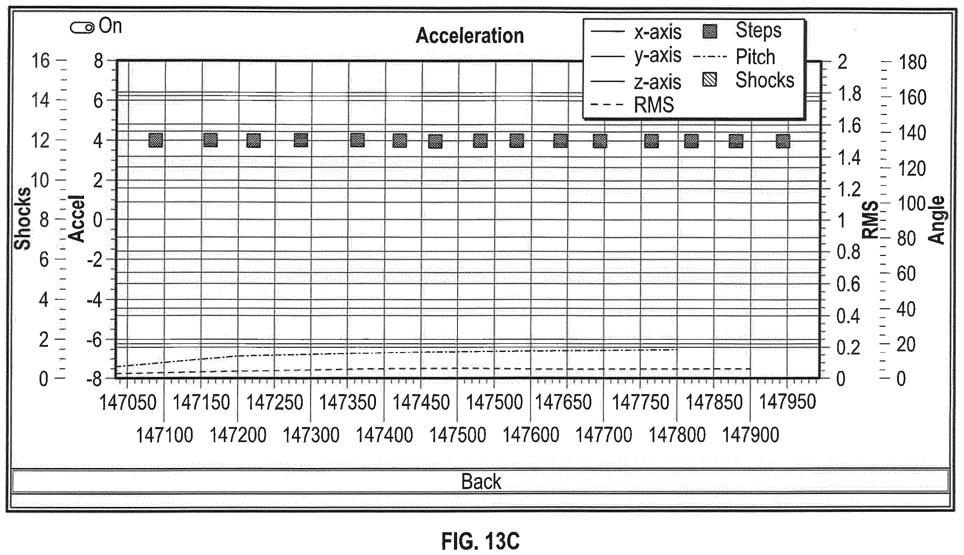

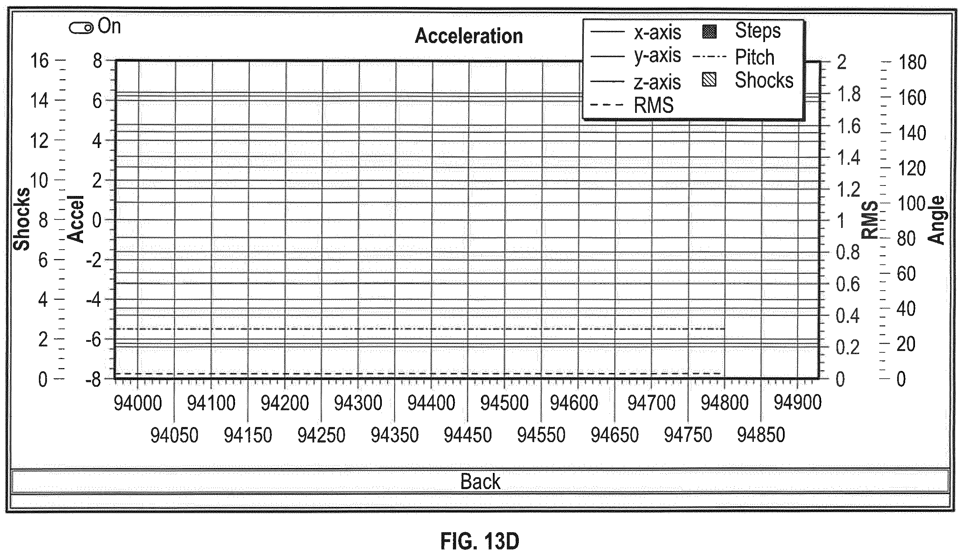

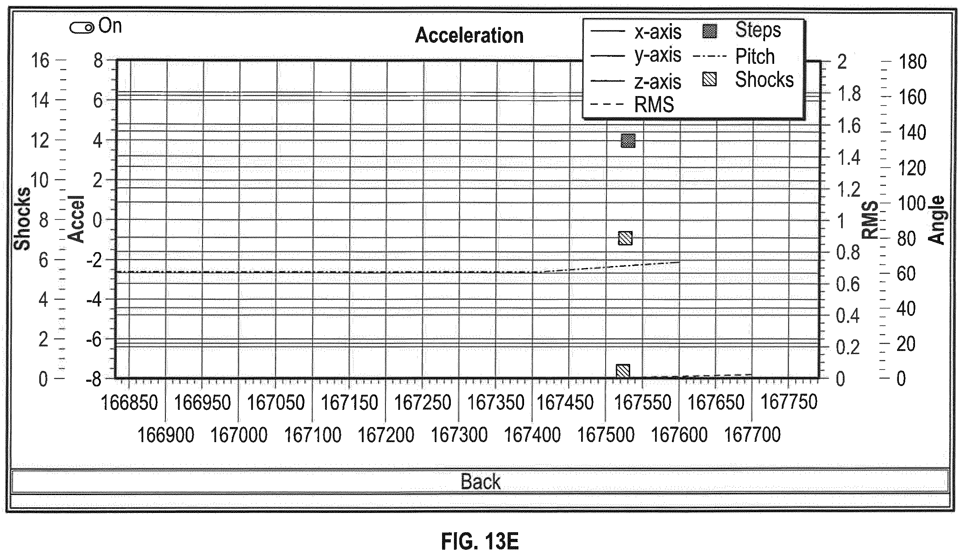

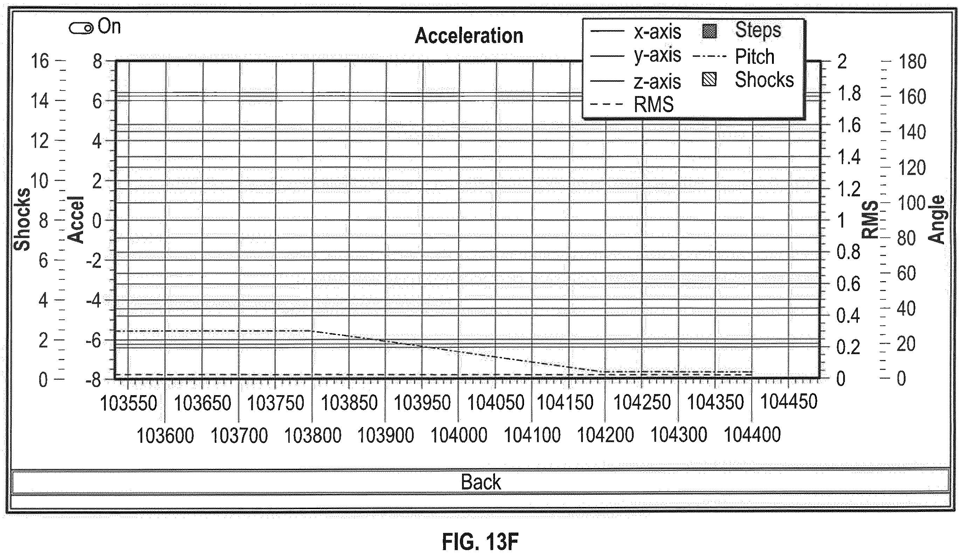

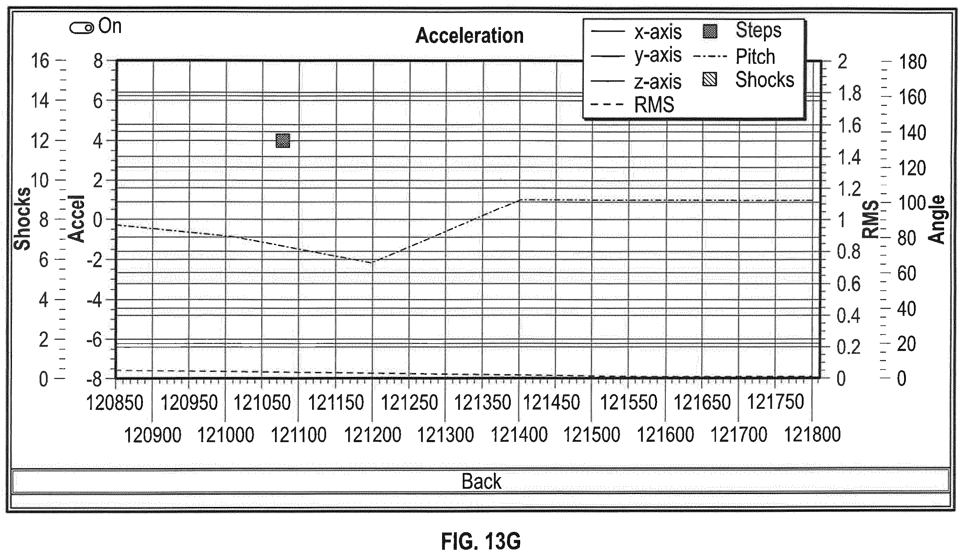

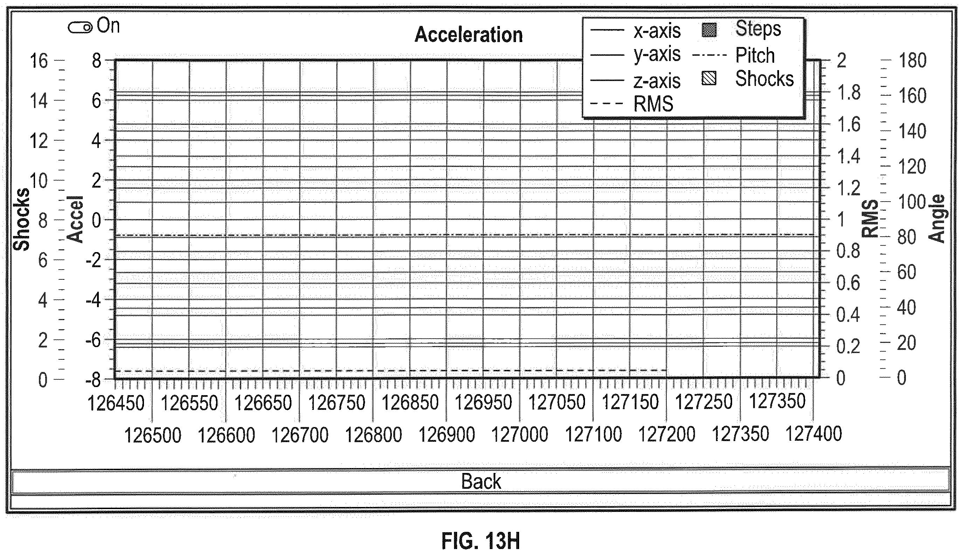

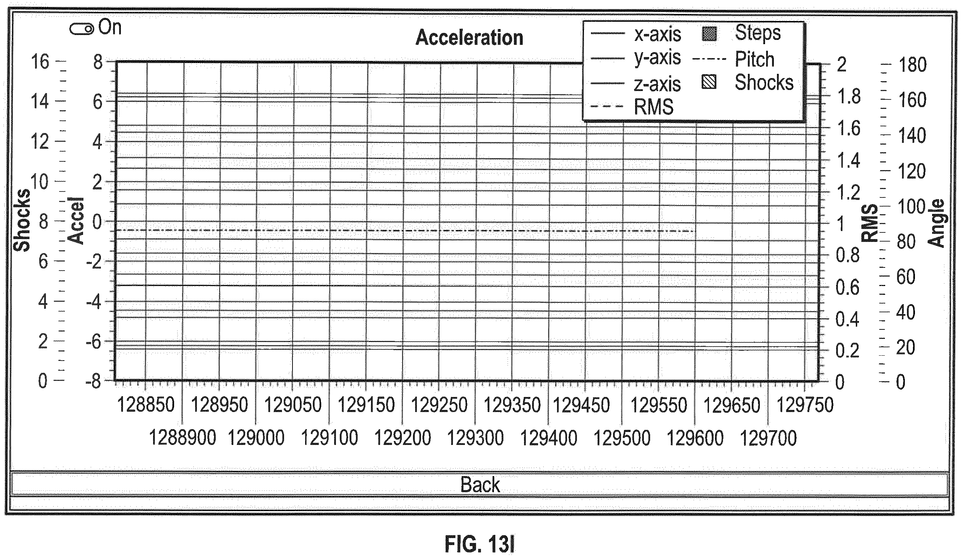

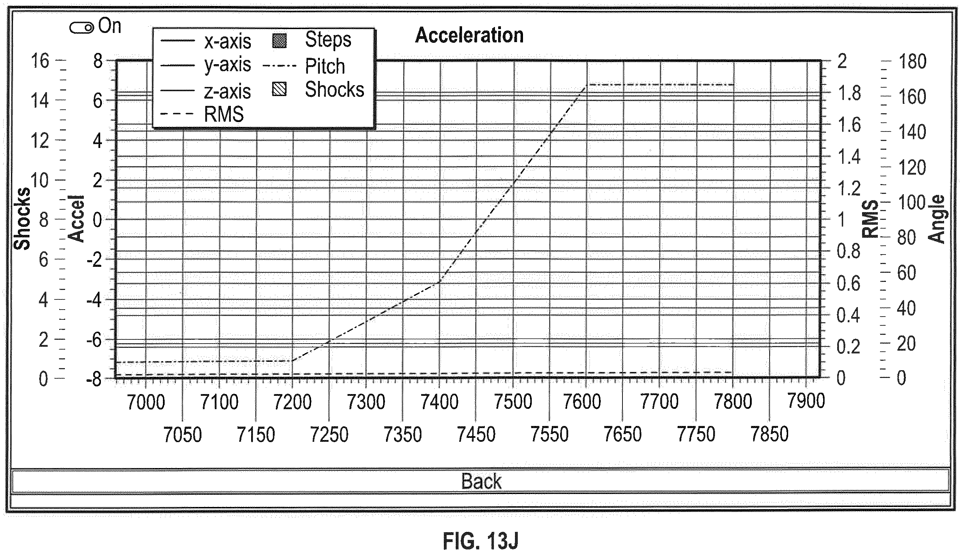

Figure 13A illustrates a plot of motion data over time collected as the TNP apparatus was moved from a stand-up position to a laying-on-back position, and Figure 13A shows a change in the pitch accordingly. Figure 13B illustrates a plot of motion data over time collected as the TNP apparatus was swung back and forth. Figure 13C illustrates a plot of motion data over time collected as the TNP apparatus was carried by a user while walking, and a timing of each of the user's step is also shown in Figure 13C. Figure 13D illustrates a plot of motion data over time collected when the TNP apparatus was positioned on an angled stand. Figure 13E illustrates a plot of motion data over time collected as the TNP apparatus was dropped suddenly and, as shown in Figure 13E, when a shock was detected. Figure 13F illustrates a plot of motion data over time collected as the TNP apparatus was moved from an angled stand to a stand-up position. Figure 13G illustrates a plot of motion data over time collected as the TNP apparatus was moved from a laying-on-back position to a laying-on-front position. Figure 13H illustrates a plot of motion data over time collected when the TNP apparatus was laying on its left side. Figure 13I illustrates a plot of motion data over time collected when the TNP apparatus was laying on its right side. Figure 13J illustrates a plot of motion data over time collected as the TNP apparatus was moved from an upright position to an upside down position. As shown Figure 13J, the pitch increased from almost 0° to 180°, indicating that the TNP apparatus was inverted.

As shown in Figures 13A-13J, the orientation of the TNP apparatus as well as movements of the TNP apparatus such as walking or shocks can be detected by the motion sensor. As discussed herein, determinations from motion data, such as the motion data plotted in Figures 13A-13J, may be used by a TNP apparatus to determine a cause of an error condition at the TNP apparatus or trigger an alarm, notification, or provide instructions, or the like.

### Other Variations

While certain embodiments have been described, these embodiments have been presented by way of example only, and are not intended to limit the scope of protection, which is defined by the appended claims. The various components illustrated in the figures may be implemented as software or firmware on a processor, controller, ASIC, FPGA, or dedicated hardware. Hardware components, such as processors, ASICs, FPGAs, and the like, can include logic circuitry.

User interface screens illustrated and described herein can include additional or alternative components. These components can include menus, lists, buttons, text boxes, labels, radio buttons, scroll bars, sliders, checkboxes, combo boxes, status bars, dialog boxes, windows, and the like. User interface screens can include additional or alternative information. Components can be arranged, grouped, displayed in any suitable order.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

The scope of the present disclosure is not intended to be limited by the specific disclosures of preferred embodiments in this section or elsewhere in this specification, but is defined by the appended claims.

## Claims

1. An apparatus (310) for applying negative pressure to a wound, the apparatus comprising:
a housing;
a pressure source (313) supported by the housing and configured to couple via a fluid flow path to a wound dressing positioned on a wound and provide negative pressure to the wound;
a controller (311) supported by the housing and configured to operate the pressure source to provide negative pressure to the wound;
an output device (314) supported by the housing and configured to provide identification data to an electronic device, the identification data being usable by the electronic device to access a label associated with the housing or one or more components supported by the housing; and
**characterized in that** the output device comprises a display that is configured to present the identification data as an optical, machine-readable representation of the identification data.

2. The apparatus of any one or more preceding claims, wherein the identification data is usable by the electronic device to access the label from a remote database via a computer network.

3. The apparatus of claim 1 or 2, wherein the optical, machine-readable representation of the identification data comprises a two-dimensional barcode.

4. The apparatus of any one or more preceding claims, wherein the output device comprises a transmitter configured to wirelessly transmit the identification data to the electronic device.

5. The apparatus of any one or more preceding claims in combination with the electronic device.

6. The apparatus of claim 5, wherein the electronic device is configured to execute an application that receives the identification data from the output device and transmits a request for the label according to the identification data.

7. The apparatus of claim 5, wherein the electronic device is configured to execute an application that receives the identification data from the output device, transmits via a computer network a request for the label according to the identification data, presents the label on a display to a user, and enables the user of the electronic device to instruct the controller to operate the pressure source to provide negative pressure to the wound dressing.

8. The apparatus of claim 7, wherein the electronic device is configured to execute an application that transmits a confirmation or a verification of presentation of the label on the display.

9. The apparatus of any one or more preceding claims, wherein the controller is configured to determine a location of the housing and automatically select the identification data from a plurality of identification data according to the location.

10. The apparatus of any one or more preceding claims, wherein the controller is configured to inactivate the pressure source until the output device provides the identification data to the electronic device.

11. The apparatus of any one or more preceding claims, wherein the electronic device comprises a mobile personal computer that is configured to communicate via a cellular communications network.

12. A method for operating a wound therapy system, the method comprising:
retrieving identification data from a memory device of a wound therapy device;
outputting the identification data from the wound therapy device to an electronic device;
receiving the identification data with the electronic device;
generating a request from the identification data with the electronic device, the request being a request to access a label associated with the wound therapy device;
transmitting the request with the electronic device via a computer network to a remote database;
receiving the label via the computer network;
outputting the label for presentation to a user of the electronic device and **characterized in that** said outputting the label comprises displaying the label on a display of the electronic device.

13. The method of claim 12, further comprising transmitting via the computer network a confirmation or a verification of said outputting.

14. The method of claim 12 or 13,
wherein said outputting the identification data comprises presenting, on a display of the negative pressure wound therapy device, the identification data as an optical, machine-readable representation of the identification data, and
wherein said receiving the identification data comprises receiving the identification data with an image sensor of the electronic device.

15. The method of any one of claims 12, 13 or 14,
wherein said outputting the identification data comprises wirelessly transmitting the identification data with a transmitter of the negative pressure wound therapy device, and
wherein said receiving the identification data comprises receiving the identification data with a receiver of the electronic device.

## Patentansprüche

1. Einrichtung (310) zum Anwenden von Unterdruck auf eine Wunde, wobei die Einrichtung umfasst:
ein Gehäuse;
eine Druckquelle (313), die vom Gehäuse getragen und dazu ausgelegt ist, über einen Fluidströmungsweg mit einem auf einer Wunde positionierten Wundverband gekoppelt zu werden und einen Unterdruck für die Wunde bereitzustellen;
eine Steuerung (311), die vom Gehäuse getragen und dazu ausgelegt ist, die Druckquelle zu betreiben, um einen Unterdruck an die Wunde bereitzustellen;
eine Ausgabevorrichtung (314), die vom Gehäuse getragen und dazu ausgelegt ist, Identifikationsdaten an eine elektronische Vorrichtung bereitzustellen, wobei die Identifikationsdaten durch die elektronische Vorrichtung nutzbar sind, um eine Kennzeichnung abzurufen, die zum Gehäuse oder einer oder mehreren vom Gehäuse getragenen Komponenten gehört; und
**dadurch gekennzeichnet, dass** die Ausgabevorrichtung eine Anzeige umfasst, die dazu ausgelegt ist, die Identifikationsdaten als optische, maschinenlesbare Darstellung der Identifikationsdaten zu präsentieren.

2. Einrichtung nach einem oder mehreren vorhergehenden Ansprüchen, wobei die Identifikationsdaten durch die elektronische Vorrichtung nutzbar sind, um über ein Computernetzwerk eine Kennzeichnung aus einer entfernten Datenbank abzurufen.

3. Einrichtung nach Anspruch 1 oder 2, wobei die optische, maschinenlesbare Darstellung der Identifikationsdaten einen zweidimensionalen Strichcode umfasst.

4. Einrichtung nach einem oder mehreren vorhergehenden Ansprüchen, wobei die Ausgabevorrichtung einen Sender umfasst, der dazu ausgelegt ist, die Identifikationsdaten drahtlos an die elektronische Vorrichtung zu senden.

5. Einrichtung nach einem oder mehreren vorhergehenden Ansprüchen in Kombination mit der elektronischen Vorrichtung.

6. Einrichtung nach Anspruch 5, wobei die elektronische Vorrichtung dazu ausgelegt ist, eine Anwendung auszuführen, die die Identifikationsdaten von der Ausgabevorrichtung empfängt und eine Anforderung der den Identifikationsdaten entsprechenden Kennzeichnung sendet.

7. Einrichtung nach Anspruch 5, wobei die elektronische Vorrichtung dazu ausgelegt ist, eine Anwendung auszuführen, die die Identifikationsdaten von der Ausgabevorrichtung empfängt, über ein Computernetzwerk eine Anforderung der den Identifikationsdaten entsprechenden Kennzeichnung sendet, die Kennzeichnung einem Benutzer auf einer Anzeige präsentiert und den Benutzer der elektronischen Vorrichtung befähigt, die Druckquelle zu betreiben, um einen Unterdruck an den Wundverband bereitzustellen.

8. Einrichtung nach Anspruch 7, wobei die elektronische Vorrichtung dazu ausgelegt ist, eine Anwendung auszuführen, die eine Bestätigung oder eine Verifizierung der Darstellung der Kennzeichnung auf der Anzeige sendet.

9. Einrichtung nach einem oder mehreren vorhergehenden Ansprüchen, wobei die Steuerung dazu ausgelegt ist, einen Standort des Gehäuses zu bestimmen und die Identifikationsdaten automatisch aus mehreren dem Standort entsprechenden Identifikationsdaten auszuwählen.

10. Einrichtung nach einem oder mehreren vorhergehenden Ansprüchen, wobei die Steuerung dazu ausgelegt ist, die Druckquelle zu deaktivieren, bis die Ausgabevorrichtung die Identifikationsdaten an die elektronische Vorrichtung bereitstellt.

11. Einrichtung nach einem oder mehreren vorhergehenden Ansprüchen, wobei die elektronische Vorrichtung einen mobilen Personal Computer umfasst, der dazu ausgelegt ist, über ein mobiles Kommunikationsnetzwerk zu kommunizieren.

12. Verfahren zum Betreiben eines Wundtherapiesystems, wobei das Verfahren umfasst:
Abfragen von Identifikationsdaten aus einer Speichervorrichtung einer Wundtherapievorrichtung;
Ausgeben der Identifikationsdaten aus der Wundtherapievorrichtung an eine elektronische Vorrichtung;
Empfangen der Identifikationsdaten mit der elektronischen Vorrichtung;
Erzeugen einer Anforderung der Identifikationsdaten mit der elektronischen Vorrichtung, wobei die Anforderung eine Anforderung ist, eine zur Wundtherapievorrichtung gehörende Kennzeichnung abzurufen;
Senden der Anforderung durch die elektronische Vorrichtung über ein Computernetzwerk an eine entfernte Datenbank;
Empfangen der Kennzeichnung über das Computernetzwerk;
Ausgeben der Kennzeichnung zur Darstellung an einen Benutzer der elektronischen Vorrichtung und **dadurch gekennzeichnet, dass** das Ausgeben der Kennzeichnung Anzeigen der Kennzeichnung auf einer Anzeige der elektronischen Vorrichtung umfasst.

13. Verfahren nach Anspruch 12, ferner umfassend Senden einer Bestätigung oder einer Verifizierung der Ausgabe über das Computernetzwerk.

14. Verfahren nach Anspruch 12 oder 13,
wobei das Ausgeben der Identifikationsdaten Präsentieren der Identifikationsdaten als optische, maschinenlesbare Darstellung der Identifikationsdaten auf einer Anzeige der Unterdruck-Wundtherapievorrichtung umfasst und
wobei das Empfangen der Identifikationsdaten Empfangen der Identifikationsdaten mit einem Bildsensor der elektronischen Vorrichtung umfasst.

15. Verfahren nach einem der Ansprüche 12, 13 oder 14, wobei das Ausgeben der Identifikationsdaten drahtloses Senden der Identifikationsdaten mit einem Sender der Unterdruck-Wundtherapievorrichtung umfasst und
wobei das Empfangen der Identifikationsdaten Empfangen der Identifikationsdaten mit einem Empfänger der elektronischen Vorrichtung umfasst.

## Revendications

1. Appareil (310) pour appliquer une pression négative à une plaie, l'appareil comprenant :
un boîtier ;
une source de pression (313) supportée par le boîtier et configurée pour se coupler par l'intermédiaire d'un chemin d'écoulement de fluide à un pansement placé sur une plaie et fournir une pression négative à la plaie ;
un contrôleur (311) supporté par le boîtier et configuré pour faire fonctionner la source de pression afin de fournir une pression négative à la plaie ;
un dispositif de sortie (314) supporté par le boîtier et configuré pour fournir des données d'identification à un dispositif électronique, les données d'identification étant utilisables par le dispositif électronique pour accéder à une étiquette associée au boîtier ou à un ou plusieurs composants supportés par le boîtier ; et
**caractérisé en ce que** le dispositif de sortie comprend un afficheur configuré pour présenter les données d'identification sous forme d'une représentation optique, lisible par machine, des données d'identification.

2. Appareil selon l'une quelconque des revendications précédentes, dans lequel les données d'identification peuvent être utilisées par le dispositif électronique pour accéder à l'étiquette à partir d'une base de données distante par l'intermédiaire d'un réseau informatique.

3. Appareil selon la revendication 1 ou la revendication 2, dans lequel la représentation optique lisible par machine des données d'identification comprend un code à barres bidimensionnel.

4. Appareil selon l'une quelconque des revendications précédentes, dans lequel le dispositif de sortie comprend un émetteur configuré pour transmettre sans fil les données d'identification au dispositif électronique.

5. Appareil selon l'une quelconque des revendications précédentes, en combinaison avec le dispositif électronique.

6. Appareil selon la revendication 5, dans lequel le dispositif électronique est configuré pour exécuter une application qui reçoit les données d'identification du dispositif de sortie et transmet une demande pour l'étiquette conformément aux données d'identification.

7. Appareil selon la revendication 5, dans lequel le dispositif électronique est configuré pour exécuter une application qui reçoit les données d'identification du dispositif de sortie, transmet, par l'intermédiaire d'un réseau informatique, une demande d'étiquette selon les données d'identification, présente l'étiquette sur un afficheur à l'attention d'un utilisateur, et permet à l'utilisateur du dispositif électronique de donner pour instruction au contrôleur d'actionner la source de pression pour fournir une pression négative au pansement.

8. Appareil selon la revendication 7, dans lequel le dispositif électronique est configuré pour exécuter une application qui transmet une confirmation ou une vérification de présentation de l'étiquette sur l'afficheur.

9. Appareil selon l'une quelconque des revendications précédentes, dans lequel le contrôleur est configuré pour déterminer un emplacement du boîtier et sélectionner automatiquement les données d'identification parmi une pluralité de données d'identification en fonction de l'emplacement.

10. Appareil selon l'une quelconque des revendications précédentes, dans lequel le contrôleur est configuré pour désactiver la source de pression jusqu'à ce que le dispositif de sortie fournisse les données d'identification au dispositif électronique.

11. Appareil selon l'une quelconque des revendications précédentes, dans lequel le dispositif électronique comprend un ordinateur personnel mobile qui est configuré pour communiquer par l'intermédiaire d'un réseau de communication cellulaire.

12. Procédé de fonctionnement d'un système de thérapie pour plaie, le procédé comprenant les étapes suivantes :
récupérer des données d'identification à partir d'un dispositif de mémoire d'un dispositif de thérapie pour plaie ;
délivrer en sortie les données d'identification depuis le dispositif de thérapie pour plaie à un appareil électronique ;
recevoir les données d'identification avec le dispositif électronique ;
générer une demande à partir des données d'identification avec le dispositif électronique, la demande étant une demande d'accès à une étiquette associée au dispositif de thérapie pour plaie ;
transmettre la demande avec le dispositif électronique par l'intermédiaire d'un réseau informatique vers une base de données distante ;
recevoir l'étiquette par l'intermédiaire du réseau informatique ;
délivrer en sortie l'étiquette pour présentation à un utilisateur du dispositif électronique, ce qui est **caractérisé en ce que** ladite sortie de l'étiquette comprend l'affichage de l'étiquette sur un afficheur du dispositif électronique.

13. Procédé selon la revendication 12, comprenant en outre de transmettre, par l'intermédiaire du réseau informatique, une confirmation ou une vérification de ladite sortie.

14. Procédé selon la revendication 12 ou la revendication 13,
dans lequel ladite sortie des données d'identification comprend la présentation, sur un afficheur du dispositif de thérapie pour plaie par pression négative, des données d'identification sous la forme d'une représentation optique lisible par machine des données d'identification, et
dans lequel ladite réception des données d'identification comprend une réception des données d'identification par un capteur d'image du dispositif électronique.

15. Procédé selon l'une quelconque des revendications 12, 13 ou 14,
dans lequel ladite sortie des données d'identification comprend la transmission sans fil des données d'identification avec un émetteur du dispositif de thérapie pour plaie par pression négative, et
dans lequel ladite réception des données d'identification comprend une réception des données d'identification avec un récepteur du dispositif électronique.
